(19) Europäisches Patentamt
European Patent Office
Office européen des brevets

(11) **EP 4 095 262 A2**

(12) **EUROPEAN PATENT APPLICATION**

(43) Date of publication:
**30.11.2022 Bulletin 2022/48**

(21) Application number: **22165085.6**

(22) Date of filing: **26.10.2018**

(51) International Patent Classification (IPC):
***C12Q 1/6869*** *(2018.01)*

(52) Cooperative Patent Classification (CPC):
(C-Sets available)
**C12Q 1/6869** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2017 JP 2017208652**

(62) Document number(s) of the earlier application(s) in accordance with Art. 76 EPC:
**18202846.4 / 3 476 946**

(71) Applicant: **SYSMEX CORPORATION**
**Kobe-shi**
**Hyogo 651-0073 (JP)**

(72) Inventors:
- **Suzuki, Kenichiro**
  **Kobe-shi, Hyogo, 651-0073 (JP)**
- **Suzuki, Seigo**
  **Kobe-shi, Hyogo, 651-0073 (JP)**
- **Watanabe, Reiko**
  **Kobe-shi, Hyogo, 651-0073 (JP)**
- **Sato, Jun**
  **Kobe-shi, Hyogo, 651-0073 (JP)**
- **Sawa, Kenichi**
  **Kobe-shi, Hyogo, 651-0073 (JP)**
- **Mino, Kenjiro**
  **Kobe-shi, Hyogo, 651-0073 (JP)**
- **Oka, Akihide**
  **Kobe-shi, Hyogo, 651-0073 (JP)**

(74) Representative: **Hoffmann Eitle**
**Patent- und Rechtsanwälte PartmbB**
**Arabellastraße 30**
**81925 München (DE)**

Remarks:
•The complete document including Reference Table(s) and the Sequence Listing(s) can be downloaded from the EPO website
•This application was filed on 29-03-2022 as a divisional application to the application mentioned under INID code 62.
•Claims filed after the date of filing of the application / after the date of receipt of the divisional application (Rule 68(4) EPC).

# (54) QUALITY EVALUATION METHOD, QUALITY EVALUATION APPARATUS, PROGRAM, STORAGE MEDIUM, AND QUALITY CONTROL SAMPLE

(57) Disclosed is a quality evaluation method performed in a genetic test for testing a gene in a sample collected from a subject, for a plurality of types of gene mutations that include a first type gene mutation and a second type gene mutation different from the first type gene mutation, and the quality evaluation method includes: preparing a quality control sample that includes a first reference gene having the first type gene mutation, and a second reference gene having the second type gene mutation; obtaining sequence information of the genes included in the quality control sample; and outputting an index for evaluation of a quality of the genetic test, based on the sequence information having been obtained.

FIG. 3

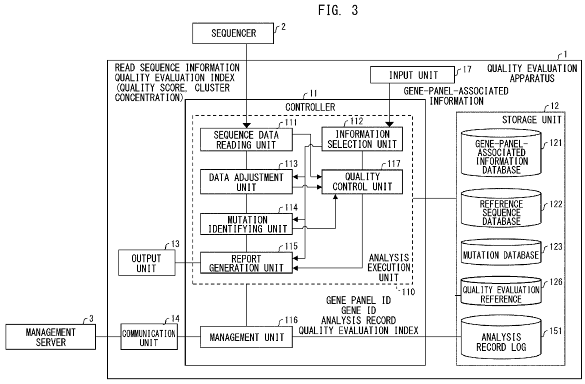




Processed by Luminess, 75001 PARIS (FR)

(Cont. next page)

(52) Cooperative Patent Classification (CPC): (Cont.)

C-Sets
**C12Q 1/6869, C12Q 2537/165, C12Q 2545/101**

## Description

CROSS REFERENCE TO RELATED APPLICATIONS

This application claims priority from prior Japanese Patent Application No. 2017-208652, filed on October 27, 2017, entitled "QUALITY EVALUATION METHOD, QUALITY EVALUATION APPARATUS, PROGRAM, STORAGE MEDIUM, AND QUALITY CONTROL SAMPLE", the entire content of which is incorporated herein by reference.

FIELD OF THE INVENTION

The present invention relates to a quality evaluation method, a quality evaluation apparatus, a program, a storage medium, and a quality control sample, which are used for a genetic test.

BACKGROUND

Development of genetic test technology in recent years enhances expectation of individualized medical care in which gene sequences of a subject are analyzed and a therapeutic method or medication is appropriately selected according to the characteristics of the subject. For example, a panel test in which abnormality in a specific gene associated with a specific disease or abnormality in an exon region that is translated into protein is analyzed by using a next-generation sequencer with high throughput, is known for analyzing gene sequences.

In Lih et al., Analytical Validation of the Next-Generation Sequencing Assay for a Nationwide Signal-Finding Clinical Trial, The Journal of Molecular Diagnostics, Vol. 19, No. 2, March 2017 (hereinafter, referred to as Non-Patent Literature 1), a quality control method for a genetic test using a next-generation sequencer is described.

However, the quality control in the genetic test field is in the trial stage, and the quality control method for a genetic test using a next-generation sequencer is not established as a standard quality control method for genetic tests. For example, when the technique disclosed in Non-Patent Literature 1 is used as a quality control method for a panel test in which a plurality of genes are to be analyzed, accuracy for quality evaluation may become low.

A main object of the present invention is to provide a quality control method, for genetic tests, performed with enhanced accuracy when a plurality of genes are to be analyzed in panel tests or the like.

SUMMARY OF THE INVENTION

In order to solve the aforementioned problem, a quality evaluation method according to one mode of the present invention is directed to a quality evaluation method performed in a genetic test for testing a gene in a sample collected from a subject, for a plurality of types of gene mutations that include a first type gene mutation and a second type gene mutation different from the first type gene mutation, and the quality evaluation method includes: preparing a quality control sample that includes a first reference gene having the first type gene mutation, and a second reference gene having the second type gene mutation; obtaining sequence information of the genes included in the quality control sample; and outputting an index for evaluation of a quality of the genetic test, based on the sequence information having been obtained.

In the above-described configuration, the quality control sample is analyzed to output an index for evaluation of a quality of a genetic test. The quality control sample includes at least one reference gene having mutation of a mutation type to be detected in the genetic test. Therefore, indexes corresponding to various mutation types can be outputted for evaluating a quality of the genetic test. Thus, quality control for a genetic test can be performed with sufficient accuracy.

A "subject" represents a human subject or a subject, which is not human, such as a mammal, an invertebrate, a vertebrate, a fungus, a yeast, a bacterium, a virus, or a plant. The embodiment herein relates to a human subject, but the concept of the present disclosure can be applied to a genome derived from an organism such as any animal or any plant other than human, and is useful in fields such as medical care, veterinary medicine, and zoological science.

A "sample" can be also referred to as a specimen, and is used so as to be synonymous with a preparation in this field. A "sample" is intended to mean any preparation obtained from a biological material (for example, individual, body fluid, cell strain, cultured tissue, or tissue section) as a supply source.

A "quality control sample" is intended to mean a preparation that is prepared for performing, for example, pretreatment for analyzing a sequence of a gene, and a process for reading sequence information by the sequencer.

"Mutation" includes substitution, deletion, or insertion of nucleotide of a gene, gene fusion, or copy number polymorphism. "Substitution" represents a phenomenon that at least one base in a gene sequence is changed to a different base. "Substitution" includes point mutation and single nucleotide polymorphism. "Deletion" and "insertion" are also referred to as "InDel (Insertion and/or Deletion)". InDel represents a phenomenon that insertion and/or deletion occurs in at least one base in a gene sequence. "Gene fusion" represents a phenomenon that the 5' side sequence of a gene binds to the 3' end side sequence of another gene due to translocation of chromosome or the like. "Copy number polymorphism" indicates that the number of copies on a genome per one cell is different among individuals. Specific examples of the copy number polymorphism include Variable Nucleotide of Tandem Repeat (VNTR), Short Tandem Repeat Polymorphism (STRP, microsatellite polymorphism), and gene amplification.

The first type gene mutation may be substitution, deletion, or insertion of nucleotide, polymorphism, gene copy number abnormalities, or gene fusion, and the second type gene mutation may be substitution, deletion, or insertion of nucleotide, polymorphism, gene copy number abnormalities, or gene fusion, which is different from the first type gene mutation.

The quality control sample may include a reference gene having at least one gene mutation for each of a plurality of mutation types to be detected in the genetic test.

A quality of the genetic test may be evaluated based on the index.

Both sequence information of a gene included in the quality control sample and sequence information of a gene included in a sample may be obtained.

The quality control sample may further include a gene that does not have mutation.

A quality of the genetic test may be evaluated based on whether or not the index satisfies a predetermined criterion.

The predetermined criterion may be different between a first gene panel used in the genetic test and a second gene panel different from the first gene panel.

Sequence information of a gene included in the quality control sample may be obtained by a sequencer, and the index may represent a quality of reading of the sequence information by the sequencer.

The index may represent an accuracy for each base in a gene sequence read by the sequencer.

The sequencer may amplify genes on a flow cell to form clusters, and the index may represent a degree to which the clusters of each gene are close to each other on the flow cell.

Sequence information of a gene included in the quality control sample may be obtained by the sequencer, and the index may represent a proportion of bases in a target region to bases having been read by the sequencer.

The index may represent a depth of sequence information, of a gene included in the quality control sample, which has been obtained.

The index may represent a variation in depth of sequence information, of a gene included in the quality control sample, which has been obtained.

A plurality of quality control samples including different genes may be prepared, and a quality control sample to be prepared may be selected from among the plurality of quality control samples.

A quality control sample that includes at least one gene which is not a target to be analyzed in the genetic test and which has mutation of the mutation type, may be prepared.

The index may be transmitted to a management server (3) connected to a plurality of test facilities.

A result of quality evaluation may be received from the management server (3).

A quality evaluation apparatus (1) according to one mode of the present invention is directed to a quality evaluation apparatus (1) for evaluating a quality of a genetic test for testing a gene in a sample collected from a subject, for at least a first type gene mutation and a second type gene mutation different from the first type gene mutation, and the quality evaluation apparatus (1) includes: a data adjustment unit (113) configured to analyze sequence information of a gene in a quality control sample that includes a first reference gene having the first type gene mutation and a second reference gene having the second type gene mutation; and a quality management unit (117) configured to generate an index for evaluation of a quality of the genetic test, based on the sequence information.

In the above-described configuration, a quality control sample is analyzed to generate an index for evaluation of a quality of a genetic test. The quality control sample includes at least one reference gene having mutation of a mutation type to be detected in the genetic test. Therefore, indexes corresponding to various mutation types can be generated for evaluating a quality of the genetic test. Thus, when the quality evaluation apparatus provides a use with an index, quality control for a genetic test can be performed with sufficient accuracy.

A program according to one mode of the present invention is directed to a quality evaluation program for a genetic test for testing a gene in a sample collected from a subject, for at least a first type gene mutation and a second type gene mutation different from the first type gene mutation, and the program causes a computer to perform: obtaining sequence information of a gene in a quality control sample that includes a first reference gene having the first type gene mutation, and a second reference gene having the second type gene mutation; and generating an index for evaluation of a quality of the genetic test, based on the sequence information having been obtained.

In the above-described configuration, the effect similar to that obtained by the quality evaluation method according to one mode of the present invention can be obtained.

A storage medium according to one mode of the present invention is directed to a computer-readable storage medium having stored therein the program according to one mode of the present invention.

A quality control sample according to one mode of the present invention is directed to a quality control sample for use in a genetic test for testing a gene in a sample collected from a subject, for at least a first type gene mutation and a second type gene mutation different from the first type gene mutation, and the quality control sample includes: a first reference gene having the first type gene mutation, and a second reference gene having the second type gene mutation.

In the above-described configuration, at least one reference gene having mutation of a mutation type to be detected in the genetic test is included. Therefore, indexes corresponding to various mutation types can be generated, by applying the quality control sample, for evaluating a quality of the genetic test.

According to one mode of the present invention, a quality control method, for a genetic test, having sufficient accuracy can be provided.

BRIEF DESCRIPTION OF THE DRAWINGS

FIG. 1 illustrates a typical exemplary application of a gene analyzing system according to one embodiment of the present invention;
FIG. 2 is a sequence diagram illustrating an example of a main process performed by the gene analyzing system;
FIG. 3 is a functional block diagram that includes an example of a software configuration of a quality evaluation apparatus;
FIG. 4 is a flow chart showing an example of a flow of a process of analyzing a gene sequence of a sample;
FIG. 5A to 5D are each a flow chart showing an example of a procedure of pretreatment for analyzing a base sequence of sample DNA by using a sequencer;
FIG. 6A to 6D illustrate an example of a quality control sample;
FIG. 7 illustrates an example of a data structure of a gene-panel-associated information database;
FIG. 8A and 8B each illustrate a specific example of a quality control sample;
FIG. 9A illustrates an example of a step (a) of fragmenting a sample;
FIG. 9B illustrates an example of a step (b) of adding index sequences and adapter sequences;
FIG. 10 illustrates an example of a hybridizing step;
FIG. 11 illustrates an example of a step of collecting DNA fragments to be analyzed;
FIG. 12 is a flow chart showing an example of a procedure of analyzing a base sequence of sample DNA by using a sequencer;
FIG. 13 illustrates an example of a step of applying DNA fragments to a flow cell;
FIG. 14 illustrates an example of a step of amplifying DNA fragments to be analyzed;
FIG. 15 illustrates an example of sequencing step;
FIG. 16 is a flow chart showing an example of a flow of analysis by the quality evaluation apparatus;
FIG. 17 illustrates an example of a file format of read sequence information;
FIG. 18A illustrates alignment performed by a data adjustment unit;
FIG. 18B illustrates an example of a format of a result of alignment performed by the data adjustment unit;
FIG. 19 illustrates an example of a structure of a reference sequence database;
FIG. 20 illustrates examples of known mutations that are incorporated into reference sequences (which do not indicate wild-type sequences) included in the reference sequence database;
FIG. 21 is a flow chart showing in detail an example of a step of alignment;
FIG. 22A illustrates an example of calculating a score;
FIG. 22B illustrates another example of calculating a score;
FIG. 23 illustrates an example of a format of a result file generated by a mutation identifying unit;
FIG. 24 illustrates an example of a structure of a mutation database;
FIG. 25 illustrates in detail an example of a structure of mutation information in the mutation database;
FIG. 26 illustrates an example of a quality evaluation index;
FIG. 27 illustrates an example of a quality evaluation index;
FIG. 28 illustrates an example of a generated report;
FIG. 29 illustrates an example of a reference gene that includes substitution mutation; and
FIG. 30 illustrates an example of a reference gene that includes fusion mutation.

DETAILED DESCRIPTION OF THE PREFERRED EMBODIMENTS

[Embodiment 1]

One embodiment of the present disclosure will be described below in detail.

(Example of application of gene analyzing system 100)

Firstly, a gene analyzing system 100 according to one embodiment of the present disclosure will be schematically described with reference to FIG. 1. FIG. 1 illustrates a typical exemplary application of the gene analyzing system 100 according to one embodiment of the present disclosure. The gene analyzing system 100 is a system for analyzing gene sequence information, and may include at least a quality evaluation apparatus 1 and a management server 3.

The gene analyzing system 100 shown in FIG. 1 is used in an analyzing system management institution 130 that entirely manages analyses performed by a test institution 120, and the test institution 120 that analyzes a sample which is provided from a medical institution 210 according to a request for analysis and provides the medical institution 210 with the analysis result.

The test institution 120 tests and/or analyzes the sample provided by the medical institution 210, generates a report based on the analysis result, and provides the medical institution 210 with the report. In the test institution 120, a sequencer 2, the quality evaluation apparatus 1, and the like are mounted. However, the test institution 120 is not limited thereto.

The analyzing system management institution 130 entirely manages the analyses performed by each test institution 120 that uses the gene analyzing system 100.

The medical institution 210 is an institution in which doctors, nurses, pharmacists, and the like perform medical practice for patients such as diagnosis, treatment, and dispensation. Examples of the medical institution 210 include hospitals, clinics, and pharmacies.

(Process in exemplary application of gene analyzing system 100)

Subsequently, a flow of a process in an exemplary application of the gene analyzing system 100 shown in FIG. 1 will be more specifically described with reference to FIG. 2. FIG. 2 is a sequence diagram illustrating an example of a main process performed by the gene analyzing system 100. The process shown in FIG. 2 is merely a part of processes performed in each institution.

<Application for use of gene analyzing system and start of use thereof>

Firstly, the test institution 120 that would like to use the gene analyzing system 100 introduces the quality evaluation apparatus 1. The test institution 120 applies for the use of the gene analyzing system 100 to the analyzing system management institution 130 (step S101).

The test institution 120 and the analyzing system management institution 130 can make a desired contract therebetween among a plurality of contract types, in advance, for the use of the gene analyzing system 100. For example, service contents provided to the test institution 120 by the analyzing system management institution 130, a method for determining a system usage fee for which the analyzing system management institution 130 bills the test institution 120, or a method for payment of the system usage fee may be selected from among a plurality of different contract types. The management server 3 of the analyzing system management institution 130 specifies the contents of the contract made with the test institution 120 according to application from the test institution 120 (step S102).

Next, the management server 3 managed by the analyzing system management institution 130 assigns a test institution ID to the quality evaluation apparatus 1 of the test institution 120 with which the analyzing system management institution 130 has made the contract, and starts providing various services (step S103).

The quality evaluation apparatus 1 receives various services from the management server 3. Examples of various services include providing of programs and information for controlling; an analysis result of gene sequences which can be outputted from the quality evaluation apparatus 1; a report based on the analysis result; and the like. Thus, the quality evaluation apparatus 1 can output, for example, the analysis result and the report which correspond to inputted gene-panel-associated information.

In many cases, a gene panel includes a set of reagents such as a primer and a probe. The analysis of the gene panel is not limited thereto, and the gene panel may be used for analysis of polymorphism such as single nucleotide polymorphism (SNP) and copy number polymorphism (CNV). The gene panel may be used for outputting information (also referred to as tumor mutation burden) associated with an amount of mutation of the entirety of genes to be analyzed, or calculating methylation frequency.

<Request for analysis to test institution 120>

In the medical institution 210, a doctor or the like collects, as appropriate, a sample such as blood and tissue of a lesion site of a subject. When a request for analyzing the collected sample is made to the test institution 120, for example, the request for analysis is transmitted via a communication terminal 5 disposed in the medical institution 210 (step S105). When a request for analyzing a sample is made to the test institution 120, the medical institution 210 transmits the request for analysis to the test institution 120 and provides the test institution 120 with a sample ID assigned to each sample. The sample ID assigned to each sample is used for associating, for example, information on a subject from which each sample is collected, and the sample with each other.

Hereinafter, an exemplary case where the medical institution 210 requests the test institution 120 to perform a panel test analysis, will be described. The panel tests include not only clinical laboratory tests but also tests for research.

When a request for a gene panel test is made by the medical institution 210, a desired gene panel may be designated. Therefore, in step S105 in FIG. 2, the request for analysis transmitted from the medical institution 210 may include gene-panel-associated information. In the description herein, the gene-panel-associated information may be any information which can be used for specifying a gene panel, and may represent, for example, a gene panel name and a name of a gene to be analyzed in the panel test.

<Analysis in test institution 120>

The quality evaluation apparatus 1 receives a request for analysis from the medical institution 210 (S106). The quality evaluation apparatus 1 receives a sample from the medical institution 210 that has transmitted the request for analysis.

The number of gene panels usable in the analysis for which the medical institution 210 makes a request to the test institution 120, is plural, and a gene group to be analyzed is determined for each gene panel. In the test institution 120, a plurality of gene panels can be selectively used according to the purpose of the analysis. That is, for a first sample provided by the medical institution 210, a first gene panel can be used in order to analyze a first gene group to be analyzed, and, for a second sample, a second gene panel can be used in order to analyze a second gene group to be analyzed.

In the present embodiment, for example, a first type gene mutation is "substitution" and a second type gene mutation is "deletion". In this case, in the genetic test to be performed for quality control, at least presence or absence of substitution and deletion, and the type are tested. In the present embodiment, a first reference gene includes a specific substitution mutation with respect to wild-type gene sequences, and a second reference gene includes a specific deletion mutation with respect to wild-type sequences of paired electrons.

The quality evaluation apparatus 1 receives, from a user, an input of gene-panel-associated information on a gene panel to be used for analyzing a sample (step S107).

In the test institution 120, pretreatment is performed for the received sample by using the gene panel, and sequencing is performed by using the sequencer 2 (step S108).

In the test institution 120, for a predetermined quality control sample corresponding to a gene panel, pretreatment is performed by using the gene panel, and sequencing is performed by using the sequencer 2 (step S108), separately from a general sample sequencing, thereby controlling accuracy.

When the quality control sample is subjected to genetic test such as pretreatment, sequencing, and sequence analysis, the result of the genetic test is used as a quality evaluation index of the panel test.

One or a plurality of quality control samples may be associated with each gene panel. For example, the quality control sample(s) corresponding to each gene panel may be prepared in advance. The quality control sample(s) may be measured alone, or may be measured together with a sample provided from the medical institution 210.

In the description herein, the quality control sample is a sample for quality control used in the genetic test in which the first type gene mutation and the second type gene mutation different from the first type gene mutation are tested. The "quality control sample" is a preparation that includes a first reference gene including the first type gene mutation and a second reference gene including the second type gene mutation.

The pretreatment may include processing from fragmenting genes such as DNA included in a sample to collecting the fragmented genes. The sequencing includes processing of reading sequences of one or a plurality of DNA fragments, to be analyzed, which are collected in the pretreatment. The sequence information read in the sequencing by the sequencer 2 is outputted as read sequence information to the quality evaluation apparatus 1.

The pretreatment may include processing from fragmenting genes such as DNA included in a sample and a quality control sample to collecting the fragmented genes.

The read sequence represents polynucleotide sequence obtained by sequencing, and represents a sequence outputted by the sequencer 2.

The sequencing includes processing of reading sequences of one or a plurality of DNA fragments, to be analyzed, which are collected in the pretreatment. The sequence information read in the sequencing by the sequencer 2 is outputted as the read sequence information to the quality evaluation apparatus 1.

The sequencer 2 may output, to the quality evaluation apparatus 1, the read sequence information that includes a quality score which is a quality evaluation index for the process step of reading the gene sequences. The sequencer 2 may output, to the quality evaluation apparatus 1, a cluster concentration that is a quality evaluation index for a process step of amplifying DNA fragments to be analyzed. The "quality score" and the "cluster concentration" will be described below.

The number of the gene panels which can be used in analysis for which the medical institution 210 makes a request to the test institution 120 is plural, and a gene group to be analyzed is determined for each gene panel. The test institution 120 can selectively use the plurality of gene panels according to the purpose of the analysis. That is, for the first sample provided by the medical institution 210, the first gene panel is used for analyzing the first gene group to be analyzed, and, for the second sample, the second gene panel can be used for analyzing a second gene group to be analyzed.

The quality evaluation apparatus 1 obtains the read sequence information from the sequencer 2 and analyzes gene sequences (step S109).

The quality control sample is also processed in the same process step as performed in the panel test for the sample from the medical institution 210, and sequence information of genes in the quality control sample is analyzed. A quality evaluation index for evaluating the quality of the panel test is generated based on the result of analyzing the quality control sample.

Next, the quality evaluation apparatus 1 evaluates the quality of the panel test based on the generated quality evaluation index (step S110). Specifically, the quality evaluation apparatus 1 can evaluate the quality of each panel test, based on a result of comparison between the evaluation criterion which is set for each quality evaluation index, and the generated quality evaluation index.

The quality evaluation apparatus 1 generates a report based on the result of the analysis in step S109, and an index generated based on the result of analyzing the quality control sample (step Sill), and transmits the generated report to the communication terminal 5 (step S112). For example, the report may include: data of an alignment result of the read sequence information: data of a result of analysis itself by the quality evaluation apparatus 1, such as data associated with identified mutation or the like; and information associated with the quality of the panel test.

The generated report may be printed in the test institution 120. For example, the test institution 120 may transmit the generated report as a paper medium to the medical institution 210.

The quality evaluation apparatus 1 of the test institution 120 that uses the gene analyzing system 100 notifies the management server 3 of gene-panel-associated information used in the analysis, information associated with the analyzed genes, analysis record, the quality evaluation index generated for the genetic test having been performed, and the like (step S114).

The management server 3 obtains a test institution ID, a gene panel ID, a gene ID, an analysis record, and the like via, for example, a network 4 from the quality evaluation apparatus 1 of each test institution 120 that uses the gene analyzing system 100. The management server 3 stores the obtained test institution ID, gene panel ID, gene ID, analysis record, and the like so as to associate them with each other (step S115).

The test institution ID is information for specifying a user who performs gene sequence analysis, and may be a user ID that is identification information assigned to each user that uses the quality evaluation apparatus 1.

The gene panel ID is identification information provided for specifying a gene panel used for analyzing a target gene. The gene panel ID assigned to the gene panel is associated with a gene panel name, a name of a company that provides the gene panel, and the like.

The gene ID is identification information provided to each gene for specifying a gene to be analyzed.

(Configuration of gene analyzing system 100)

The gene analyzing system 100 analyzes gene sequence information, and includes at least the quality evaluation apparatus 1 and the management server 3. The quality evaluation apparatus 1 is connected to the management server 3 via the network 4 such as an intranet and the Internet.

(Sequencer 2)

The sequencer 2 is a base sequence analyzing device used for reading a base sequence of a gene included in a sample.

The sequencer 2 according to the present embodiment is preferably a next-generation sequencer that performs sequencing using a next-generation sequencing technique, or a third-generation sequencer. The next-generation sequencer is a series of base sequence analyzing devices which are being developed in recent years, and has a significantly improved analytical capability by performing, in a flow cell, parallel processing of a large amount of single DNA molecules or DNA templates which have been clonally amplified.

Sequencing technology usable in the present embodiment may be sequencing technology in which a plurality of reads are obtained by reading the same region multiple times (deep sequencing).

Examples of the sequencing technology usable in the present embodiment include sequencing technology, such as ionic semiconductor sequencing, pyrosequencing, sequencing-by-synthesis which uses a reversible dye terminator, sequencing-by-ligation, and sequencing by probe ligation of oligonucleotide, which can obtain multiple reads per one run and is based on sequencing principle other than the Sanger's method.

The sequencing primer used in the sequencing is not particularly limited, and is set as appropriate based on the sequence suitable for amplifying a target region. A reagent used in the sequencing may be also suitably selected according to the sequencing technology and the sequencer 2 to be used. A procedure from the pretreatment to the sequencing will be described below by using a specific example.

(Configuration of quality evaluation apparatus 1)

FIG. 3 illustrates an example of a configuration of the quality evaluation apparatus 1. The quality evaluation apparatus 1 includes: a controller 11 that obtains the read sequence information read by the sequencer 2 and gene-panel-associated information on gene panels that include a plurality of genes to be analyzed; and an output unit 13 that outputs a result of analysis of the read sequence information based on the gene-panel-associated information which is obtained by the controller 11. The quality evaluation apparatus 1 can be configured by using a computer. For example, the controller 11 is implemented by a processor such as a CPU, and a storage unit 12 is implemented by a hard disk drive.

In the storage unit 12, a program for sequence analysis, a program for generating a single reference sequence, and the like are stored. The output unit 13 includes a display, a printer, a speaker, and the like. An input unit 17 includes a keyboard, a mouse, a touch sensor, and the like. A device, which functions as both the input unit and the output unit, such as a touch panel having a touch sensor and a display integrated with each other may be used. A communication unit 14 is an interface that allows the controller 11 to communicate with an external device.

The quality evaluation apparatus 1 includes: the controller 11 that comprehensively controls the components of the quality evaluation apparatus 1; the storage unit 12 that stores various data used by an analysis execution unit 110; the output unit 13, the communication unit 14, and the input unit 17. The controller 11 includes the analysis execution unit 110 and a management unit 116. The analysis execution unit 110 includes a sequence data reading unit 111, an information selection unit 112, a data adjustment unit 113, a mutation identifying unit 114, and a report generation unit 115. In the storage unit 12, a gene-panel-associated information database 121, a reference sequence database 122, a mutation database 123, and an analysis record log 151 are stored.

The quality evaluation apparatus 1 generates, even when a gene panel to be used is changed for each analysis, a report that includes a result of analysis corresponding to the gene panel having been used. A user using the gene analyzing system 100 is allowed to analyze the result of a panel test by a common analysis program to generate a report regardless of a type of the gene panel. Therefore, when the panel test is performed, a bothersome operation such as selecting an analysis program to be used for each gene panel, and performing specific setting for the analysis program for each gene panel to be used is omitted, thereby improving usability for a user.

When a user of the quality evaluation apparatus 1 inputs gene-panel-associated information from the input unit 17, the information selection unit 112 refers to the gene-panel-associated information database 121, and controls an algorithm of an analysis program so as to execute analysis of a gene to be analyzed by the analysis program according to the inputted gene-panel-associated information.

In the description herein, the gene-panel-associated information may be any information for specifying a gene panel used for measurement by the sequencer 2, and represents, for example, a gene panel name, a name of a gene to be analyzed in the gene panel, and a gene panel ID.

The information selection unit 112 changes an analysis algorithm so as to perform analysis corresponding to a gene to be analyzed in the gene panel indicated by the gene-panel-associated information, based on the gene-panel-associated information which is inputted by the input unit 17.

The information selection unit 112 outputs an instruction based on the gene-panel-associated information to at least one of the data adjustment unit 113, the mutation identifying unit 114, and the report generation unit 115. By using this configuration, the quality evaluation apparatus 1 can output a result of the analysis of the read sequence information, based on the inputted gene-panel-associated information.

That is, the information selection unit 112 is a functional block for performing control so as to obtain gene-panel-associated information on gene panels that include a plurality of genes to be analyzed, and causing the output unit 13 to output a result of analysis of the read sequence information, based on the obtained gene-panel-associated information.

When genes included in various samples are analyzed by a user that performs the panel test, various gene panels are used according to gene groups, to be analyzed, for each sample.

That is, the quality evaluation apparatus 1 can obtain first read sequence information read by using the first gene panel for analyzing, from the first sample, the first gene group to be analyzed, and second read sequence information read by using the second gene panel for analyzing, from the second sample, the second gene group to be analyzed.

Even when various combinations of genes to be analyzed are analyzed by using various gene panels, the quality evaluation apparatus 1 can appropriately output results of analyses obtained by analyzing the read sequence information since the quality evaluation apparatus 1 includes the information selection unit 112.

That is, a user merely selects gene-panel-associated information without setting an analysis program used for analyzing the read sequence information and performing analysis for each gene to be analyzed, whereby a result of analysis of each piece of the read sequence information can be appropriately outputted.

For example, when the information selection unit 112 outputs, to the data adjustment unit 113, an instruction based on the gene-panel-associated information, the data adjustment unit 113 performs, for example, an alignment process based on the gene-panel-associated information.

According to the gene-panel-associated information, the information selection unit 112 makes an instruction for limiting the reference sequence (reference sequence in which wild-type genome sequence and mutation sequence are incorporated) used by the data adjustment unit 113 in mapping of the read sequence information, only to the reference sequence associated with a gene corresponding to the gene-panel-associated information.

In this case, since the gene-panel-associated information has already been reflected on the result of the process by the data adjustment unit 113, the information selection unit 112 need not output an instruction based on the gene-panel-associated information, to the mutation identifying unit 114 that performs a process subsequent to the process performed by the data adjustment unit 113.

For example, when the information selection unit 112 outputs, to the mutation identifying unit 114, an instruction based on the gene-panel-associated information, the mutation identifying unit 114 preforms a process in which the gene-panel-associated information is reflected.

For example, according to the gene-panel-associated information, the information selection unit 112 makes an instruction for limiting a region of the mutation database 123 to which the mutation identifying unit 114 refers, only to mutation associated with a gene corresponding to the gene-panel-associated information. Thus, gene-panel-associated information is reflected on the result of the process by the mutation identifying unit 114.

(Flow of process of analyzing gene sequence of sample)

A flow of a process of analyzing gene sequences of a sample and a quality control sample will be described with reference to FIG. 4. FIG. 4 is a flow chart showing an example of a flow of a process of analyzing gene sequences of a sample.

Firstly, in step S31 in FIG. 4, pretreatment for analyzing a sequence of a gene to be analyzed is performed. The pretreatment includes a process from fragmenting genes such as DNA included in a sample and a quality control sample to collecting the fragmented genes. When the sample provided by the medical institution 210 is, for example, a tissue and blood, a process of extracting genes (for example, DNA) from the tissue or the blood is also included.

Next, in step S32, sequences of genes included in the sample and the quality control sample having been subjected to the pretreatment are read by the sequencer 2.

Step S32 is, specifically, a step of reading sequences of one or a plurality of DNA fragments, to be analyzed, which have been collected after the pretreatment. The read sequence information includes the gene sequence which is read in this step. One or a plurality of DNA fragments, to be analyzed, which have been collected after the pretreatment may be also referred to as "library".

Subsequently, when the quality control sample is measured, the quality evaluation apparatus 1 analyzes the read gene sequence and specifies presence or absence of mutation in the sequence, a position of the mutation, a type of the mutation, and the like in step S33. By the read gene sequence being analyzed, the detected mutation is identified.

Subsequently, in step S34, the quality evaluation apparatus 1 generates a quality evaluation index for evaluating the quality of the panel test. The quality evaluation apparatus 1 may evaluate the quality of the panel test having been performed, based on the generated quality evaluation index.

Finally, the quality evaluation apparatus 1 generates a report that includes a result of the analysis such as information associated with the mutation identified in step S33, and information, representing the quality of the panel test, such as the quality evaluation index generated in step S34. The generated report is provided to the medical institution 210.

(Pretreatment)

Next, a procedure of the pretreatment in step S31 shown in FIG. 4 will be described with reference to the flow shown in FIG. 5. FIG. 5 is a flow chart showing an example of a procedure of pretreatment for analyzing a base sequence of sample DNA by using the sequencer 2.

When DNA is extracted from each of the sample and the quality control sample to perform sequence analysis, DNA is firstly extracted from the sample that includes genes to be analyzed, and the quality control sample corresponding to the gene panel to be used (step 300 in FIG. 5A).

In this case, the DNA derived from the sample and the DNA derived from the quality control sample are each subjected to the process of step S301 and the subsequent steps.

The DNA extracted from the quality control sample is subjected to the same process as for the DNA extracted from the sample, whereby a quality evaluation index useful for evaluating the quality of the sequence analysis in the panel test can be generated.

The usage of the quality control sample is not limited thereto. For example, as shown in FIG. 5B, DNA of only the quality control sample may be extracted in step 300a, and subjected to the process of step S301 and the subsequent steps.

Alternatively, as shown in FIG. 5C, a quality control sample that includes mutation and a quality control sample that does not include mutation are prepared as quality control samples, and DNA may be extracted therefrom (step 300b).

By comparison between a result of analysis of DNA derived from the quality control sample that includes mutation and a result of analysis of DNA derived from the quality control sample that does not include mutation, a quality evaluation index useful for evaluating the quality of the sequence analysis in the panel test can be generated.

Furthermore, as shown in FIG. 5D, DNA may be extracted from each of a sample that includes genes to be analyzed, a quality control sample that includes mutation, and a quality control sample that does not include mutation (step 300c). The sample that includes genes to be analyzed may be a combination of a blood sample and a tumor cell sample.

In the process of step S301 and the subsequent steps, DNA derived from the sample and DNA derived from the quality control sample may be mixed to perform the process of step S301 and the subsequent steps without individually processing the DNA derived from the sample and the DNA derived from the quality control sample. Thus, in all the process of step S301 and the subsequent steps, the conditions for both of the samples are the same, whereby the quality evaluation index can be more accurately generated. A part of the lanes in the flow cell used for the sequencer 2 need not be used only for DNA fragments prepared from the quality control sample. Thus, the limited number of lanes can be effectively used for DNA fragments derived from a sample that includes genes to be analyzed.

In this case, (1) a reagent for appropriately fragmenting a reference gene included in a quality control sample and a gene to be analyzed in the panel test to prepare a library, and (2) a reagent that contains RNA baits for appropriately capturing the DNA fragments, respectively, after the reference gene included in the quality control sample and the gene to be analyzed in the panel test are fragmented, are preferably utilized.

(Quality control sample)

According to one embodiment, the quality control sample is a composition containing a plurality of reference genes. The quality control sample can be prepared by a plurality of reference genes being mixed. A reagent obtained by the reference genes being mixed and stored in a single container can be provided as the quality control sample to a user. A plurality of reference genes that are stored in separate containers may be provided in the form of a kit as the quality control sample to a user. The quality control sample may be in the form of a solution or may be in a solid state (powder). When the quality control sample is provided in the form of solution, an aqueous solvent, such as water and TE buffer, known to a person skilled in the art can be used as the solvent.

The quality control sample will be described with reference to FIG. 6. FIG. 6 illustrates an example of a quality control sample.

FIG. 6A illustrates a list of genes which can be genes to be analyzed in the panel test using the gene panel. One or a plurality of the genes in the list are associated as genes to be analyzed in the gene panel (see data 121B in FIG. 7).

FIG. 6B illustrates an example of types of mutations to be detected in the panel test. Types of mutations to be detected are "single nucleotide variant (SNV)", "Insertion" and "Deletion" (in FIG. 6B, indicated as "InDel"), "copy number polymorphism (CNV)", and "Fusion".

A quality control sample A1 corresponding to a gene panel A includes at least two of a reference gene including SNV, a reference gene including Insertion, a reference gene including Deletion, a reference gene including CNV, and a reference gene including Fusion. For example, the quality control sample A1 includes, as the reference genes, a partial sequence of a gene A including "SNV" with respect to a wild-type gene, and a partial sequence of a gene B including "Insertion" with respect to a wild-type gene.

FIG. 6D illustrates an example of output of a result of analysis of the quality control sample and a result of analysis of the genetic test using the gene panel A. In this example, as a result of the analysis of the gene panel A, SNV of each of GNA11, AKT1, and PIK3CA, Long insertion and Long deletion of EGFR, SLC34A2-ROS1 fusion gene, CCDC6-RET fusion gene, gene amplification of MET, gene amplification of MYC-N, and gene amplification of MYC-C are detected.

The quality control sample of the gene panel A includes a reference gene that includes SNV of GNA11, a reference gene that includes SNV of AKT1, a reference gene that includes SNV of PIK3CA, a reference gene that includes Long insertion of EGFR, a reference gene that includes Long deletion of EGFR, a reference gene that includes SLC34A2-ROS1 fusion sequence, a reference gene that includes CCDC6-RET fusion sequence, a reference gene that includes gene amplification of MET, a reference gene that includes gene amplification of MYC-N, and a reference gene that includes gene amplification of MYC-C. In this example, the quality control sample includes 10 types of reference genes. However, the quality control sample is not limited to this example.

The first reference gene and the second reference gene included in the quality control sample may be different DNA molecules, or may ligate each other. When the first reference gene and the second reference gene ligate each other, the sequence of the first reference gene and the sequence of the second reference gene may directly ligate each other, or a spacer sequence may intervene between the sequence of the first reference gene and the sequence of the second reference gene.

The spacer sequence is preferably a sequence which is less likely to be included in a specimen used for the genetic test. For example, the space sequence may be a sequence in which only a plurality (for example, 100) of adenine bases are consecutive.

The reference gene may be a gene included in a gene panel to be analyzed, or a gene which is not included in the gene panel to be analyzed. The reference gene may be a gene of a biological species for which the genetic test is to be performed, or a gene of a different biological species. For example, when the genetic test is performed for the human, the reference gene may be a gene of an animal other than the human, a plant, or bacteria.

A method for synthesizing the reference gene is not particularly limited. For example, the reference gene can be synthesized by a known DNA synthesizer. A gene, derived from an organism, which serves as a template is amplified by PCR and purified, whereby the reference gene may be obtained. PCR amplification is performed by using, as a template, a reference gene synthesized by a DNA synthesizer and purification is performed, whereby the reference gene may be obtained.

The length of the reference gene is not particularly limited. For example, the length of the reference gene may be greater than or equal to the length of 50 nucleotides. When amplification by PCR is performed, amplification can be advantageously performed with ease such that the length of the reference gene is less than or equal to the length of 2000 nucleotides. When the reference gene is synthesized by a DNA synthesizer, up to several kbp of the reference gene can be synthesized.

The concentration of the reference genes in the quality control sample is not particularly limited. For example, the concentration of the reference genes can be approximately the same as a DNA concentration in the specimen.

The reference gene in the quality control sample may be single-stranded or double-stranded. The reference gene may be linear or cyclic.

Hereinafter, one example of preparation of the quality control sample will be specifically described.

(1) Preparation of reference gene including substitution mutation

A reference gene having a sequence represented by sequence number 1 is synthesized by a known DNA synthesizer. The synthesized DNA is amplified by PCR by using a commercially available reagent that contains DNA polymerase, dNTPs, and buffer. FIG. 29 shows the sequence represented by sequence number 1. This sequence is a sequence of exon 20 of PIK3CA gene, and includes substitution (A3140G) of G for A at position 3140 of the wild-type PIK3CA gene (as to A3140G, see U. S. Patent No. 8026053. The content of U. S. Patent No. 8026053 is incorporated herein by reference). The sequence represented by sequence number 1 has 476 mer length, and substitution mutation of A3140G is located at position 204. A coding sequence of the wild-type PIK3CA gene is represented by sequence number 2.

The amplification product is subjected to agarose gel electrophoresis, and a band portion near 500 bp is cut out. The gel having been cut out is purified in a fixed method. After the purification, DNA is quantified, and is diluted by a TE buffer to a desired concentration, whereby the reference gene having the sequence represented by sequence number 1 is obtained.

(2) Preparation of reference gene including fusion mutation

A reference gene having a sequence represented by sequence number 3 is synthesized by a known DNA synthesizer. The synthesized DNA is amplified by PCR by using a commercially available reagent that contains DNA polymerase, dNTPs, and buffer. FIG. 30 shows the sequence represented by sequence number 3. The sequence represented by sequence number 3 is a partial sequence of EML4-ALK fusion gene. In the sequence represented by sequence number 3, the sequence of positions 1 to 500 is derived from EML4 gene, and the sequence of positions 501 to 1000 is derived from ALK gene (see FIG. 30). EML4-ALK fusion gene is registered in GenBank Accession No.

AB663645.1. The sequence represented by sequence number 3 is the sequence of positions 1158 to 2157 in GenBank Accession No. AB663645.1.

Similarly to (1) described above, a reference gene having the sequence represented by sequence number 3 is obtained.

(3) Preparation of quality control sample including reference gene

Reference DNA molecules having the sequence represented by sequence number 1 and reference DNA molecules having the sequence represented by sequence number 3 are mixed at a desired concentration, to prepare a quality control sample. The quality control sample is mixed with a specimen to prepare a sample for sequence analysis.

(4) Analysis

The quality of gene panel test is evaluated by using the prepared sample for sequence analysis by a next-generation sequencer (for example, NextSeq500 manufactured by Illumina, Inc.). In the gene panel, a plurality of genes that include PIK3CA genes and EML4-ALK fusion genes are target genes. The genomic DNA derived from the specimen in the sample for sequence analysis, and the reference gene are subjected to the pretreatment (fragmentation, DNA concentration, PCR amplification using tag primer, and the like) and the sequence analysis, to obtain sequence information of the target genes. In the sequence analysis, an index for quality control is obtained, and the quality of a result of analysis of the target gene is evaluated based on the index of sequence analysis of the reference DNA molecules. A user is allowed to determine reliability of the result of analysis of the gene to be analyzed, based on the result of the quality evaluation.

In the example described above, in (3), the quality control sample and the specimen are mixed. However, each of the quality control sample and the specimen may be separately subjected to the sequence analysis without mixing them.

When the panel test using the same gene panel is repeated, the same quality control sample may be repeatedly used. As indicated by data 121D in FIG. 7, a plurality of kinds of quality control samples including different types of mutations and different reference genes may be prepared as a plurality of quality control samples corresponding to each gene panel.

When a plurality of quality control samples having different combinations of reference genes are selectively used for each panel test, on a weekly basis, or on a monthly basis, the quality evaluation index for evaluating the quality of the process of detecting mutation in the panel test can be generated by detecting mutations of the increased number of kinds of reference genes. Therefore, the comprehensiveness of the quality control in the panel test is improved.

For example, FIG. 8 shows a quality control sample A and a quality control sample B that are quality control samples corresponding to a gene panel A. A reference gene a1, a reference gene a2, and a reference gene a3 included in the quality control sample A are changed to a reference gene b1, a reference gene b2, and a reference gene b3 in the quality control sample B, respectively.

Next, as shown in FIG. 9A, the sample (genomic DNA derived from the specimen, and/or reference gene) is fragmented so as to have a length with which the sequencer 2 reads the sequence (step S301 in FIG. 5). The sample DNA can be fragmented by a known method such as ultrasonication and a process using a reagent for fragmenting nucleic acid. The obtained DNA fragment (nucleic acid fragment) can have a length of, for example, several tens of bp to several hundreds of bp.

Next, as shown in FIG. 9B, adapter sequences corresponding to a sequence protocol and a type of the sequencer 2 to be used are added to both ends (3 '-end and 5 '-end) of the DNA fragment obtained in step S301 (step S302 in FIG. 5). Although this process step is indispensable when the sequencer 2 is a sequencer manufactured by Illumina, Inc. or a device which adopts the same mode of the sequencer as manufactured by Illumina, Inc., this process step may be omitted in some cases when another type of the sequencer 2 is used.

The adapter sequence is a sequence used for executing the sequencing in the following process steps. According to one embodiment, in Bridge PCR method, the adapter sequence can be a sequence which is hybridized with oligo DNA immobilized on a flow cell.

In one mode, as shown in the upper part in FIG. 9B, adapter sequences (for example, adapter 1 sequence and adapter 2 sequence in FIG. 9) may be added directly to both ends of the DNA fragment. The adapter sequences can be added to the DNA fragment by using a known method in this technical field. For example, the DNA sequence may be blunted and ligated with the adapter sequences.

The adapter sequences can be added to the DNA fragment by using a known method in this technical field. For example, the DNA fragment may be blunted and ligated with an index sequence, and, thereafter, may be further ligated with the adapter sequences.

Next, as shown in FIG. 10, a biotinylated RNA bait library is hybridized with the DNA fragment to which the adapter sequences have been added (step S303 in FIG. 5).

The biotinylated RNA bait library is formed from a biotinylated RNA (hereinafter, referred to as RNA bait) which is hybridized with a gene to be analyzed. The RNA bait may have any length. For example, long oligo RNA bait having about 120 bp may be used in order to enhance specificity.

In the panel test using the sequencer 2 according to the present embodiment, multiple genes (for example, greater than or equal to 100 genes) are to be analyzed.

A reagent used in the panel test includes a set of RNA baits corresponding to the multiple genes, respectively. When the panel is different, the number and the kinds of genes to be tested are different, whereby a set of RNA baits that are contained in the reagent used in the panel test is different. When a gene different from a gene to be analyzed is used as a reference gene, a bait that binds to the reference gene need to be prepared.

As shown in FIG. 11, DNA fragments to be analyzed are collected (step S304 in FIG. 5). Specifically, as indicated in the upper part in FIG. 11, streptavidin magnetic beads obtained by streptavidin and magnetic beads binding to each other are mixed with the DNA fragments with which the biotinylated RNA bait library has been hybridized.

Thus, as indicated in the mid-part in FIG. 11, the streptavidin portion of the streptavidin magnetic bead and the biotin portion of the RNA bait bind to each other. As indicated in the lower part in FIG. 11, the streptavidin magnetic beads are collected by a magnet, and the fragments (that is, DNA fragments which are not to be analyzed) which are not hybridized with the RNA baits are removed by washing.

Thus, the DNA fragments which are hybridized with the RNA baits, that is, the DNA fragments to be analyzed can be selected and concentrated. The sequencer 2 reads nucleic acid sequences of the DNA fragments selected by using a plurality of RNA baits, thereby obtaining a plurality of read sequences.

(Reading of read sequence by sequencer 2)

Next, the procedure of step S32 in FIG. 4 will be described based on the flow shown in FIG. 12 with reference where appropriate to FIG. 13 to FIG. 15. FIG. 12 is a flow chart showing an example of a procedure of analyzing a base sequence of sample DNA by using the sequencer 2.

As shown in FIG. 13 from the left part to the center part, the streptavidin magnetic beads and the RNA baits are removed from the concentrated DNA fragments, and amplification by PCR method is performed to complete the pretreatment.

Firstly, as indicated in the right part in FIG. 13, the sequences of the amplified DNA fragments are applied to a flow cell (step S305 in FIG. 12).

Subsequently, as shown in FIG. 14, the DNA fragments to be analyzed are amplified on the flow cell by the Bridge PCR method (step S306 in FIG. 12).

That is, two different kinds of adapter sequences (for example, adapter 1 sequence and adapter 2 sequence in FIG. 14) are added, in the above-described pretreatment, to both ends of the DNA fragment (for example, Template DNA in FIG. 14) to be analyzed ("1" in FIG. 14), and the DNA fragment is separated into single strands, and the adapter 1 sequence on the 5’ end side is immobilized on the flow cell ("2" in FIG. 14).

The adapter 2 sequence on the 3’ end side is immobilized on the flow cell in advance, and the adapter 2 sequence on the 3’ end side of the DNA fragment binds to the adapter 2 sequence on the 3’ end side on the flow cell to form a bridged state, thereby forming a bridge ("3" in FIG. 14).

When DNA elongation by DNA polymerase is caused in this state ("4" in FIG. 14), and denaturation is caused, two single-stranded DNA fragments are obtained ("5" in FIG. 14).

Forming of the bridge, DNA elongation, and denaturation as described above are repeatedly performed in order, respectively, whereby multiple single-stranded DNA fragments are locally amplified and immobilized to form a cluster ("6" to "10" in FIG. 14).

As shown in FIG. 15, the single-stranded DNA that forms the cluster is used as a template, and the sequence is read by sequencing-by-synthesis (step S307 in FIG. 12).

Firstly, to the single-stranded DNA (the upper left part in FIG. 15) immobilized on the flow cell, DNA polymerase and dNTP which is labeled with fluorescence and has the 3’ end side blocked are added (the upper center part in FIG. 15), and a sequence primer is further added thereto (the upper right part in FIG. 15).

The sequence primer may be designed, for example, to be hybridized with a part of the adapter sequence. In other words, the sequence primer may be designed so as to amplify the DNA fragment derived from the sample DNA, and, when an index sequence is added, the sequence primer may be further designed so as to amplify the index sequence.

After the sequence primer is added, one base elongation is caused, by the DNA polymerase, for dNTP which is labeled with fluorescence and has 3’ end blocked. Since the dNTP having 3’ end side blocked is used, polymerase reaction stops when one base elongation has been caused. The DNA polymerase is removed (the right center part in FIG. 15), and laser light is applied to the single-stranded DNA elongated by one base (the lower right part in FIG. 15) to excite fluorescent substance binding to the base, and a photograph of light generated at this time is taken and recorded (lower left part in FIG. 15).

The photograph is taken for each of fluorescent colors corresponding to A, C, G, T, respectively while a wavelength filter is changed in order to determine four kinds of bases by using a fluorescence microscope. After all the photographs have been obtained, bases are determined from the photograph data. Fluorescent substance and the protecting group that blocks the 3' end side are removed, and the subsequent polymerase reaction is caused. This flow is set as one cycle, and the second cycle, the third cycle, and so on are repeatedly performed, whereby sequencing over the entirety of the length can be performed.

In the above-described manner, the length of the chain which can be analyzed reaches 150 bases×2, and analysis can be performed in units which are much less than those for a picotiter plate. Therefore, due to high density, a huge amount of sequence information corresponding to 40 to 200 Gb can be obtained in one analysis.

(c. Gene panel)

The gene panel used for reading the read sequence by the sequencer 2 represents an analysis kit for analyzing a plurality of targets to be analyzed in one run as described above. According to one embodiment, the gene panel can be an analysis kit for analyzing a plurality of gene sequences associated with a specific disease.

In the description herein, the term "kit" represents a packaging that includes a container (for example, bottle, plate, tube, and dish) that contains a specific material therein. The kit preferably includes an instruction for use of each material. In the description herein, according to the aspect of kit, "include (is included)" represents a state of being included in any of individual containers that form the kit. The kit can be a package in which a plurality of different compositions are packaged into one, and the mode of the compositions can be as described above. In the case of solution form, the solution may be contained in the container.

In the kit, one container may contain a material A and a material B in a mixed manner, or the material A and the material B may be contained in separate containers, respectively. The "instruction" indicates the procedure of applying the components in the kit to treatment and/or diagnosis. The "instruction" may be written or printed on paper or another medium. Alternatively, the "instruction" may be stored in an electronic medium such as a magnetic tape, a computer-readable disc or tape, and a CD-ROM. The kit may also include a container in which diluent, solvent, washing liquid, or another reagent is stored therein. The kit may also include equipment necessary for applying the kit to treatment and/or diagnosis.

In one embodiment, the gene panel may include one or more of the quality control sample, reagents such as a reagent for fragmenting nucleic acid, a reagent for ligation, washing liquid, and PCR reagent (dNTP, DNA polymerase or the like), and magnetic beads, as described above. The gene panel may also include one or more of oligonucleotide for adding an adapter sequence to fragmented DNA, oligonucleotide for adding an index sequence to fragmented DNA, the RNA bait library, and the like.

In particular, the index sequence included in each gene panel can be a sequence, specific to the gene panel, for identifying the gene panel. The RNA bait library included in each gene panel may be a library, specific to the gene panel, which includes a RNA bait corresponding to each test gene of the gene panel.

(Sequence data reading unit 111, data adjustment unit 113, and mutation identifying unit 114)

Subsequently, the sequence data reading unit 111, the data adjustment unit 113, and the mutation identifying unit 114 of the analysis execution unit 110 will be described based on the flow of the process shown in FIG. 16 with reference where appropriate to FIG. 17 to FIG. 25. FIG. 16 is a flow chart showing an example of a flow of analysis by the quality evaluation apparatus 1. The process shown in FIG. 16 corresponds to step S109 shown in FIG. 2 and step S33 shown in FIG. 4.

<Sequence data reading unit 111>

Firstly, in step S11 shown in FIG. 16, the sequence data reading unit 111 reads read sequence information provided by the sequencer 2.

The read sequence information is data representing a base sequence read by the sequencer 2. The sequencer 2 performs sequencing of multiple nucleic acid fragments obtained by using a specific gene panel, and reads the sequence information therein, and provides the quality evaluation apparatus 1 therewith as the read sequence information.

In one mode, the read sequence information may include the quality score of each base in the sequence as well as the sequence having been read. Both the read sequence information obtained by subjecting, to the sequencer 2, the FFPE sample collected from a lesion site of a subject and the read sequence information obtained by subjecting, to the sequence 2, blood sample of the subject are inputted to the quality evaluation apparatus 1.

FIG. 17 illustrates an example of a file format of the read sequence information. In the example shown in FIG. 17, the read sequence information includes a sequence name, a sequence, and a quality score. The sequence name may be, for example, a sequence ID assigned to the read sequence information outputted by the sequencer 2. The sequence represents a base sequence read by the sequencer 2. The quality score represents the probability of incorrect base assignment performed by the sequencer 2. Any base sequence quality score (Q) is represented by the following equation.

$$Q=-10\log_{10}E$$

In this equation, E represents an estimated value of the probability of incorrect base assignment. The greater the value of Q is, the lower the probability of the error is. The less the value of Q is, the greater a portion of the read which cannot be used is.

False-positive mutation assignment increases, and the accuracy of the result may be lowered. The "false-positive" means that the read sequence is determined as having mutation although the read sequence does not have true mutation to be determined.

"Positive" means that the read sequence has true mutation to be determined, and "negative" means that the read sequence does not have mutation to be determined. For example, if the quality score is 20, the probability of error is 1/100. Therefore, this means that the accuracy (also referred to as "basecall accuracy") for each base in the gene sequence having been read is 99%.

<Data adjustment unit 113>

Subsequently, in step S12 in FIG. 16, the data adjustment unit 113 performs alignment of the read sequence of each nucleic acid fragment which is included in read sequence information, based on the read sequence information read by the sequence data reading unit 111.

FIG. 18A illustrates alignment performed by the data adjustment unit 113. The data adjustment unit 113 refers to the reference sequence (reference sequence information) stored in the reference sequence database 122, and performs mapping of the read sequence of each nucleic acid fragment, to the reference sequence to be compared with the read sequence information, thereby performing the alignment. In one mode, a plurality of kinds of the reference sequences corresponding to the genes, respectively, to be analyzed are stored in the reference sequence database 122.

The data adjustment unit 113 performs alignment for both the read sequence information obtained by subjecting, to the sequencer 2, the FFPE sample collected from a lesion site of a subject, and the read sequence information obtained by subjecting, to the sequencer 2, a blood sample of the subject.

FIG. 18B illustrates an example of a format of a result of alignment performed by the data adjustment unit 113. The format of the result of alignment is not particularly limited, and may be any format that can specify the read sequence, the reference sequence, and the mapping position. As shown in FIG. 18B, the format may include reference sequence information, a read sequence name, position information, mapping quality, and a sequence.

The reference sequence information represents, for example, the reference sequence name (reference sequence ID) in the reference sequence database 122, and the sequence length of the reference sequence. The read sequence name is information that represents the name (read sequence ID) of each read sequence for which the alignment has been performed. The position information represents the position (Leftmost mapping position) on the reference sequence at which the leftmost base of the read sequence has been mapped. The mapping quality is information that represents the quality of mapping corresponding to the read sequence. The sequence is information that represents the base sequence (for example, ...GTAAGGCACGTCATA) corresponding to each read sequence.

FIG. 19 illustrates an example of a structure of the reference sequence database 122. As shown in FIG. 19, the reference sequence database 122 stores reference sequences (for example, genome sequences of chromosomes #1 to 23) representing wild-type sequences, and reference sequences in which the known mutations are incorporated in the wild-type sequences.

Metadata representing the gene-panel-associated information is added to each reference sequence in the reference sequence database 122. For example, the gene-panel-associated information which is to be added to each reference sequence can directly or indirectly indicate the gene, to be analyzed, corresponding to each reference sequence.

In one embodiment, the information selection unit 112 may perform control such that, when the data adjustment unit 113 obtains a reference sequence from the reference sequence database 122, the data adjustment unit 113 refers to the inputted gene-panel-associated information and the metadata of each reference sequence, and selects a reference sequence corresponding to the gene-panel-associated information.

For example, in one mode, the information selection unit 112 may control the data adjustment unit 113 so as to select a reference sequence corresponding to a gene, to be analyzed, which is specified by the inputted gene-panel-associated information. Thus, the data adjustment unit 113 performs mapping merely on the reference sequence associated with the gene panel having been used, thereby improving efficiency of the analysis.

In another embodiment, the information selection unit 112 need not perform the above-described control. In this case, the information selection unit 112 merely controls the mutation identifying unit 114 or the report generation unit 115 as described below.

FIG. 20 illustrates examples of known mutations that are incorporated into reference sequences (which do not indicate wild-type sequences) included in the reference sequence database 122. The known mutations are mutations registered in an external database (for example, COSMIC, ClinVar, or the like), and, as shown in FIG. 20, the chromosome positions, the gene names, and the mutations are specified. In the example shown in FIG. 20, mutations of amino acid are specified. However, mutations of nucleic acid may be specified. The types of the mutations are not particularly limited, and the mutations may be various mutations such as substitution, insertion, and deletion or the mutation may be a mutation in which a sequence of a part of another chromosome or reverse complement sequence is bound.

FIG. 21 is a flow chart showing in detail an example of a step of alignment in step S12 shown in FIG. 16. In one mode, the alignment in step S12 shown in FIG. 16 is performed in steps S401 to S405 shown in FIG. 21.

In step S401 shown in FIG. 21, the data adjustment unit 113 selects, from among the read sequences of nucleic acid fragments which are included in the read sequence information obtained by the sequence data reading unit 111, a read sequence which has not been subjected to alignment, and compares the selected read sequence with a reference sequence obtained from the reference sequence database 122. In step S402, the data adjustment unit 113 specifies a position, on the reference sequence, at which the degree of matching with the read sequence satisfies a predetermined criterion. The degree of matching is a value that represents a degree of matching to which the obtained read sequence information and the reference sequence match with each other, and represents, for example, the number or proportion of bases that match each other.

In one mode, the data adjustment unit 113 calculates a score representing the degree of matching between the read sequence and the reference sequence. The score that represents the degree of matching may be, for example, a percentage (percentage identity) of the matching between the two sequences. For example, the data adjustment unit 113 specifies positions at which bases of the read sequence and bases of the reference sequence are the same, and obtains the number of the positions, and divides the number of the positions at which the bases are the same, by the number of bases (the number of bases in the comparison window) of the read sequence compared with the reference sequence, to calculate the percentage.

FIG. 22A illustrates an example of calculating a score. In one mode, at the position shown in FIG. 22A, the score representing the degree of matching between the read sequence R1 and the reference sequence is 100% because 13 bases among 13 bases of the read sequence match with the bases of the reference sequence. The score representing the degree of matching between the read sequencer R2 and the reference sequence is 92.3% because 12 bases among 13 bases of the read sequence match with the bases of the reference sequence.

In a case where the score representing the degree of matching between the read sequence and the reference sequence is calculated, the data adjustment unit 113 may calculate the score such that, when the read sequence includes a predetermined mutation (for example, insertion·deletion (InDel: Insertion/Deletion)) with respect to the reference sequence, the score is less than that calculated in the normal calculation.

In one mode, for a read sequence that includes at least one of insertion and deletion with respect to the reference sequence, the data adjustment unit 113 may correct the score by, for example, multiplying the score calculated in the above-described normal calculation, by a weighting factor according to the number of bases corresponding to the insertion·deletion. The weighting factor W may be calculated as, for example, W={1-(1/100)×(the number of bases corresponding to insertion·deletion)}.

FIG. 22B illustrates another example of calculating a score. In one mode, at the positions shown in FIG. 22B, the score representing the degree of matching between the read sequence R3 and the reference sequence is 88% in the normal calculation because 15 bases among 17 bases of the read sequence (* representing deletion is calculated as one base) match with the bases of the reference sequence, and the corrected score is 88%×0.98=86%. The score representing the degree of matching between the read sequence R4 and the reference sequence is 81% in the normal calculation because 17 bases among 21 bases of the read sequence match with the bases of the reference sequence, and the corrected score is 81%×0.96=77.8%.

The data adjustment unit 113 calculates the score representing the degree of matching while changing the mapping position of the read sequence with respect to each reference sequence, thereby specifying a position on the reference sequence at which the degree of matching with the read sequence satisfies a predetermined criterion. At this time, an algorithm known in this technical field, such as dynamic programming, the FASTA method, and the BLAST method, may be used.

Returning to FIG. 21, subsequently, when the degree of matching with the read sequence satisfies the predetermined criterion at a single position on the reference sequence (NO in step S203), the data adjustment unit 113 performs alignment of the read sequence at the position, and, when the degree of matching with the read sequence satisfies the predetermined criterion at a plurality of positions on the reference sequence (YES in step S403), the data adjustment unit 113 performs alignment of the read sequence at the position at which the degree of matching is highest (step S404).

When alignment of all the read sequences included in the read sequence information obtained by the sequence data reading unit 111 has not been performed (NO in step S405), the data adjustment unit 113 returns the process to step S401. When alignment of all the read sequences included in the read sequence information has been performed (YES in step S405), the process step of step S12 is ended.

< Mutation identifying unit 114>

Subsequently, returning to FIG. 16, in step S13, the mutation identifying unit 114 compares the sequence (alignment sequence) of the reference sequence with which the read sequence obtained from the sample collected from the lesion site of the subject has been aligned, with the sequence (so-called alignment sequence) of the reference sequence with which the read sequence obtained from a blood sample of the subject has been aligned.

In step S14 shown in FIG. 16, a difference between both the alignment sequences is extracted as mutation. For example, at the same positions of the same genes to be analyzed, the alignment sequence derived from the blood specimen is ATCGA, and the alignment sequence derived from tumor tissue is ATCCA, the mutation identifying unit 114 extracts a difference of G and C as mutation.

In one mode, the mutation identifying unit 114 generates a result file based on the extracted mutation. FIG. 23 illustrates an example of a format of a result file generated by the mutation identifying unit 114. The format may be, for example, based on Variant Call Format (VCF).

As shown in FIG. 23, the result file contains position information, reference base, and mutation base for each extracted mutation. The position information represents a position on the reference genome, and includes, for example, chromosome number and the position on the chromosome. The reference base represents the reference base (such as A, T, C, G) at the position represented by the position information. The mutation base represents a base of the reference base which is present after the mutation. The reference base is a base, on the alignment sequence, derived from the blood specimen. The mutation base is a base, on the alignment sequence, derived from the tumor tissue.

In FIG. 23, the mutation in which the reference base is C and the mutation base is G, is an example of substitution mutation, the mutation in which the reference base is C and the mutation base is CTAG, is an example of insertion mutation, and the mutation in which the reference base is TCG and the mutation base is T is an example of deletion mutation. Mutation in which the mutation base is G]17:198982], ]13:123456]T, C[2:321682[, or [17:198983[A, is an example of the mutation in which a sequence of a part of another chromosome or reverse complement sequence is bound.

Returning to FIG. 16, subsequently, in step S15, the mutation identifying unit 114 searches the mutation database 123. In step S16, the mutation identifying unit 114 refers to the mutation information in the mutation database 123, and adds annotation to mutation included in the result file, to identify the mutation.

FIG. 24 illustrates an example of a structure of the mutation database 123. The mutation database 123 is, for example, configured based on an external database such as COSMIC or ClinVar. In one mode, metadata related to the gene-panel-associated information is added to each piece of the mutation information in the database. In the example shown in FIG. 24, a gene ID of a gene to be analyzed is added as metadata to each piece of the mutation information in the database.

FIG. 25 illustrates in detail an example of a structure of mutation information in the mutation database 123. As shown in FIG. 25, in one mode, the mutation information included in the mutation database 123 may include mutation ID, mutation position information (for example, "CHROM" and "POS"), "REF", "ALT", and "Annotation". The mutation ID is an identifier for identifying the mutation.

Among the mutation position information, "CHROM" represents the chromosome number, and "POS" represents a position on the chromosome number. "REF" represents a base in the wild-type, and "ALT" represents a base that is present after the mutation. "Annotation" represents information associated with the mutation. "Annotation" may be, for example, information representing mutation of amino acid such as "EGFR C2573G" or "EGFR L858R". For example, "EGFR C2573G" represents mutation in which cysteine at the 2573-th residue of protein "EGFR" is substituted by glycine.

As in the above-described example, "Annotation" of the mutation information may be information for converting mutation based on the base information to mutation based on the amino acid information. In this case, the mutation identifying unit 114 can convert the mutation based on the base information to the mutation based on the amino acid information, according to the information of "Annotation" which has been referred to.

The mutation identifying unit 114 searches the mutation database 123 by using, as a key, information (for example, base information corresponding to mutation position information and mutation) for specifying the mutation included in the result file. For example, the mutation identifying unit 114 may search the mutation database 123 by using, as a key, information of any of "CHROM", "POS", "REF", and "ALT". When the mutation extracted by comparison between the alignment sequence derived from the blood specimen and the alignment sequence derived from the lesion site is registered in the mutation database 123, the mutation identifying unit 114 identifies the mutation as a mutation in the sample, and adds annotation (for example, "EGFR L858R", "BRAF V600E", or the like) to the mutation included in the result file.

(Report generation unit 115)

The report generation unit 115 generates a report based on the information outputted by the mutation identifying unit 114 and the gene-panel-associated information provided by the information selection unit 112 (corresponding to step Sill in FIG. 2 and step S35 in FIG. 4). The information in the generated report includes gene-panel-associated information and information associated with the identified mutation.

The report generation unit 115 selects information to be included in the report, based on the gene-panel-associated information provided by the information selection unit 112, and eliminates, from the report, the information which has not been selected. Alternatively, the information selection unit 112 may control the report generation unit 115 so as to select gene-associated information corresponding to the gene-panel-associated information inputted via the input unit 17, as information to be included in the report, and eliminate, from the report, the information which has not been selected.

(Output unit 13)

The report generated by the report generation unit 115 may be transmitted as data to the communication terminal 5 installed in the medical institution 210, through the output unit 13, as a result of analysis of the read sequence information (corresponding to step S112 in FIG. 2). Alternatively, the report may be transmitted to a printer (not shown) connected to the quality evaluation apparatus 1 and printed by the printer, and the report may be thereafter transmitted as a paper medium from the test institution 120 to the medical institution 210.

(Quality evaluation index)

Examples of the quality evaluation index obtained by measuring the quality control sample are as follows.

- Index (i): quality evaluation index representing the quality of reading read sequence information by the sequencer 2
- Index (ii): quality evaluation index representing a proportion of bases read by the sequencer 2 to bases included in a plurality of genes to be analyzed
- Index (iii): quality evaluation index representing the depth of read sequence information
- Index (iv): quality evaluation index representing variation in depth of read sequence information
- Index (v): quality evaluation index indicating whether or not all the mutations in each reference gene included in the quality control sample have been detected

Index (i) may include
index (i-1): quality score, and
index (i-2): cluster concentration.

The above-described quality evaluation index will be described with reference to FIG. 26 to FIG. 28.

Index (i-1): quality score

The quality score is an index representing accuracy for each base in the gene sequence read by the sequencer 2.

For example, when the read sequence information is outputted as FASTQ file from the sequencer 2, the quality score is also included in the read sequence information (see FIG. 17). The quality score is described above in detail, and the description thereof is omitted.

Index (i-2): cluster concentration

The sequencer 2 locally amplifies and immobilizes multiple single-stranded DNA fragments on the flow cell to form a cluster (see 9 in FIG. 14). An image of the cluster group on the flow cell is taken by using a fluorescence microscope, and fluorescent colors (that is, fluorescences having different wavelengths) corresponding to A, C, G, T, respectively, are detected, to read the sequence. The cluster density is an index representing a degree to which the clusters of each gene formed on the flow cell are close to each other when the sequencing is performed.

For example, in a case where the cluster density is excessively high, and the clusters are excessively close to each other or overlap each other, the contrast of the taken image of the flow cell, that is, the S/N ratio is lowered, whereby focusing by the fluorescence microscope is less likely to be easily performed. Therefore, fluorescence cannot be accurately detected. As a result, the sequence cannot be accurately read.

Index (ii): quality evaluation index representing a proportion of bases in a target region read by the sequencer 2, to bases read by the sequencer 2

The index indicates how many bases in the target region have been read, among bases (also including bases other than those in the target region) read by the sequencer 2, and can be calculated as a ratio between the total number of bases in the target region and the total number of bases having been read.

Index (iii): quality evaluation index representing the depth of read sequence information

The index is an index based on the total number of pieces of the read sequence information obtained by reading the bases included in a gene to be analyzed, and can be calculated as a ratio between the total number of bases, among the bases having been read, having depths which are greater than or equal to a predetermined value, and the total number of bases having been read.

The depth represents the total number of pieces of the read sequence information having been read for one base.

FIG. 26 shows a graph representing the depth for each base having been read in a case where T base represents the entire length of the gene to be analyzed and t1 base represents the base in the read region. In the graph, the horizontal axis represents the position of each base, and the vertical axis represents the depth of each base. In the example shown in FIG. 26, the total number of bases in the region in which the depth is greater than or equal to a predetermined value (for example, 100), in the t1 base in the region having been read, is (t2+t3) bases. In this case, the index (iii) is generated as a value of (t2+t3)/t1.

Index (iv): quality evaluation index representing a variation in depth of the read sequence information

The index is an index representing the uniformity of the depth. When the number of pieces of the read sequence information having been read in a certain portion among the region having been read is extremely great, uniformity of the depth is low. When the read sequence information is relatively uniform over the region having been read, the uniformity of the depth is high. The uniformity of the depth is not limited thereto. For example, the uniformity can be represented as numbers by using the interquartile range (IQR). The greater the IQR is, the lower the uniformity is. The less the IQR is, the higher the uniformity is.

Index (v): quality evaluation index indicating whether or not all the mutations in each reference gene included in the quality control sample have been detected

The index is an index indicating that the mutation in each reference gene included in the quality control sample has been detected and accurately identified. The mutation (see the cell for "Variant") in each reference gene included in a quality control sample A shown in FIG. 27 is a known mutation. Whether or not the position of the mutation, the type of the mutation, and the like have been accurately identified, is determined and the result is used as the quality evaluation index.

FIG. 28 illustrates an example of a report generated by the report generation unit 115. In the upper left portion of the report indicated in the example, "patient ID" representing the subject ID, "sex of patient", "name of disease of patient", "name of doctor in charge" representing the name of a doctor in charge of the subject in the medical institution 210, and "name of institution" representing the name of the medical institution are indicated.

Below these items, the gene panel name "A panel" is indicated as the gene-panel-associated information. The quality evaluation index "QC index" obtained from the process using the quality control sample, the result of analysis thereof, and the like is outputted in the report.

In the report, in the cells for "detected gene mutation and associated medication", information associated with the mutation identified by the mutation identifying unit 114 and the list associated with the medication are included.

When the quality evaluation index is less than a predetermined criterion, the detected gene mutation may be marked with"*". In addition thereto or instead thereof, a comment for indicating that reliability is low can be added.

The present disclosure is not limited to the above-described embodiments. Numerous modifications can be made without departing from the scope of the appended claims. An embodiment in which techniques disclosed in different embodiments are combined with each other as appropriate may be also included in the technical scope of the present disclosure.

DESCRIPTION OF THE REFERENCE CHARACTERS

| | |
|---|---|
| 1 | quality evaluation apparatus |
| 2 | sequencer |
| 3 | management server |
| 4 | network |
| 11 | controller |
| 13 | output unit |
| 100 | gene analyzing system |
| 117 | quality control unit |
| 121 | gene-panel-associated information database |
| 122 | reference sequence database |
| 124 | medication database |
| 125 | reference database |
| 126 | quality evaluation reference |

SEQUENCE LISTING

<110> Sysmex Corporation

<120> Quality control method for genetic test

<130> 17-056JP

<160> 3

<170> PatentIn version 3.5

<210> 1
<211> 476
<212> DNA
<213> artificial

<220>
<223> artificial sequence

<400> 1

```
gtttcaggag atgtgttaca aggcttatct agctattcga cagcatgcca atctcttcat      60
aaatcttttc tcaatgatgc ttggctctgg aatgccagaa ctacaatctt ttgatgacat     120
tgcatacatt cgaaagaccc tagccttaga taaaactgag caagaggctt tggagtattt     180
catgaaacaa atgaatgatg cacgtcatgg tggctggaca acaaaaatgg attggatctt     240
ccacacaatt aaacagcatg cattgaactg aaagataact gagaaaatga aagctcactc     300
tggattccac actgcactgt taataactct cagcaggcaa agaccgattg cataggaatt     360
gcacaatcca tgaacagcat tagatttaca gcaagaacag aaataaaata ctatataatt     420
taaataatgt aaacgcaaac agggtttgat agcacttaaa ctagttcatt tcaaaa         476
```

<210> 2
<211> 3412
<212> DNA
<213> Homo sapiens

<400> 2

atgcctccaa gaccatcatc aggtgaactg tggggcatcc acttgatgcc cccaagaatc 60
ctagtggaat gtttactacc aaatggaatg atagtgactt tagaatgcct ccgtgaggct 120
acattagtaa ctataaagca tgaactattt aaagaagcaa gaaaataccc tctccatcaa 180
cttcttcaag atgaatcttc ttacattttc gtaagtgtta cccaagaagc agaaagggaa 240
gaattttttg atgaaacaag acgactttgt gatcttcggc tttttcaacc atttttaaaa 300
gtaattgaac cagtaggcaa ccgtgaagaa aagatcctca atcgagaaat tggttttgct 360
atcggcatgc cagtgtgcga atttgatatg gttaaagatc ctgaagtaca ggacttccga 420
agaaatattc ttaatgtttg taaagaagct gtggatctta gggatcttaa ttcacctcat 480
agtagagcaa tgtatgtcta tccgccacat gtagaatctt caccagagct gccaaagcac 540
atatataata aattggatag aggccaaata atagtggtga tttgggtaat agtttctcca 600
aataatgaca agcagaagta tactctgaaa atcaaccatg actgtgtgcc agaacaagta 660
attgctgaag caatcaggaa aaaaactaga agtatgttgc tatcatctga acaattaaaa 720
ctctgtgttt tagaatatca gggcaagtac attttaaaag tgtgtggatg tgatgaatac 780
ttcctagaaa aatatcctct gagtcagtat aagtatataa gaagctgtat aatgcttggg 840
aggatgccca atttgaagat gatggctaaa gaaagccttt attctcaact gccaatggac 900
tgttttacaa tgccatctta ttccagacgc atttccacag ctacaccata tatgaatgga 960
gaaacatcta caaaatccct ttgggttata aatagagcac tcagaataaa aattctttgt 1020
gcaacctacg tgaatctaaa tattcgagac attgacaaga tttatgttcg aacaggtatc 1080
taccatggag gagaaccctt atgtgacaat gtgaacactc aaagagtacc ttgttccaat 1140
cccaggtgga atgaatggct gaattatgat atatacattc ctgatcttcc tcgtgctgct 1200
cgactttgcc tttccatttg ctctgttaaa ggccgaaagg gtgctaaaga ggaacactgt 1260
ccattggcat ggggaaatat aaacttgttt gattacacag acactctagt atctggaaaa 1320
atggctttga atctttggcc agtacctcat ggattagaag atttgctgaa ccctattggt 1380
gttactggat caaatccaaa taaagaaact ccatgcttag agttggagtt tgactggttc 1440
agcagtgtgg taaagttccc agatatgtca gtgattgaag agcatgccaa ttggtctgta 1500
tcccgagaag caggatttag ctattcccac gcaggactga gtaacagact agctagagac 1560
aatgaattaa gggaaaatga caaagaacag ctcaaagcaa tttctacacg agatcctctc 1620
tctgaaatca ctgagcagga gaaagatttt ctatggagtc acagacacta ttgtgtaact 1680
atccccgaaa ttctacccaa attgcttctg tctgttaaat ggaattctag agatgaagta 1740
gcccagatgt attgcttggt aaaagattgg cctccaatca aacctgaaca ggctatggaa 1800
cttctggact gtaattaccc agatcctatg gttcgaggtt ttgctgttcg gtgcttggaa 1860
aaatatttaa cagatgacaa actttctcag tatttaattc agctagtaca ggtcctaaaa 1920
tatgaacaat atttggataa cttgcttgtg agatttttac tgaagaaagc attgactaat 1980

caaaggattg ggcacttttt cttttggcat ttaaaatctg agatgcacaa taaaacagtt 2040
agccagaggt ttggcctgct tttggagtcc tattgtcgtg catgtgggat gtatttgaag 2100
cacctgaata ggcaagtcga ggcaatggaa aagctcatta acttaactga cattctcaaa 2160
caggagagga aggatgaaac acaaaaggta cagatgaagt ttttagttga gcaaatgagg 2220
cgaccagatt tcatggatgc cctacagggc ttgctgtctc ctctaaaccc tgctcatcaa 2280
ctaggaaacc tcaggcttaa agagtgtcga attatgtctt ctgcaaaaag gccactgtgg 2340
ttgaattggg agaacccaga catcatgtca gagttactgt ttcagaacaa tgagatcatc 2400
tttaaaaatg gggatgattt acggcaagat atgctaacac ttcaaattat tcgtattatg 2460
gaaaatatct ggcaaaatca aggtcttgat cttcgaatgt taccttatgg ttgtctgtca 2520
atcggtgact gtgtgggact tattgaggtg gtgcgaaatt ctcacactat tatgcaaatt 2580
cagtgcaaag gcggcttgaa aggtgcactg cagttcaaca gccacacact acatcagtgg 2640
ctcaaagaca agaacaaagg agaaatatat gatgcagcca ttgacctgtt tacacgttca 2700
tgtgctggat actgtgtagc taccttcatt ttgggaattg gagatcgtca caatagtaac 2760
atcatggtga aagacgatgg acaactgttt catatagatt ttggacactt tttggatcac 2820
aagaagaaaa aatttggtta taaacgagaa cgtgtgccat ttgttttgac acaggatttc 2880
ttaatagtga ttagtaaagg agcccaagaa tgcacaaaga caagagaatt tgagaggttt 2940
caggagatgt gttacaaggc ttatctagct attcgacagc atgccaatct cttcataaat 3000
cttttctcaa tgatgcttgg ctctggaatg ccagaactac aatcttttga tgacattgca 3060
tacattcgaa agaccctagc cttagataaa actgagcaag aggctttgga gtatttcatg 3120
aaacaaatga atgatgcaca tcatggtggc tggacaacaa aaatggattg gatcttccac 3180
acaattaaac agcatgcatt gaactgaaag ataactgaga aaatgaaagc tcactctgga 3240
ttccacactg cactgttaat aactctcagc aggcaaagac cgattgcata ggaattgcac 3300
aatccatgaa cagcattaga tttacagcaa gaacagaaat aaaatactat ataatttaaa 3360
taatgtaaac gcaaacaggg tttgatagca cttaaactag ttcatttcaa aa 3412

<210> 3
<211> 1000
<212> DNA
<213> artificial

<220>
<223> artificial sequence

<400> 3

gttattgatg actccaatga gcatatgctt actgtatggg actggcagag gaaagcaaaa 60
ggagcagaaa taaagacaac aaatgaagtt gttttggctg tggagtttca cccaacagat 120
gcaaatacca taattacatg cggtaaatct catattttct tctggacctg gagcggcaat 180
tcactaacaa gaaaacaggg aatttttggg aaatatgaaa agccaaaatt tgtgcagtgt 240
ttagcattct tggggaatgg agatgttctt actggagact caggtggagt catgcttata 300
tggagcaaaa ctactgtaga gcccacacct gggaaaggac ctaaaggtgt atatcaaatc 360
agcaaacaaa tcaaagctca tgatggcagt gtgttcacac tttgtcagat gagaaatggg 420
atgttattaa ctggaggagg gaaagacaga aaaataattc tgtgggatca tgatctgaat 480
cctgaaagag aaatagagtt tagtgcttca agggccaggc tgccaggcca tgttgcagct 540
gaccacccac ctgcagtgta ccgccggaag caccaggagc tgcaagccat gcagatggag 600
ctgcagagcc ctgagtacaa gctgagcaag ctccgcacct cgaccatcat gaccgactac 660
aaccccaact actgctttgc tggcaagacc tcctccatca gtgacctgaa ggaggtgccg 720
cggaaaaaca tcaccctcat tcggggtctg ggccatggcg cctttgggga ggtgtatgaa 780
ggccaggtgt ccggaatgcc caacgaccca agccccctgc aagtggctgt gaagacgctg 840
cctgaagtgt gctctgaaca ggacgaactg gatttcctca tggaagccct gatcatcagc 900
aaattcaacc accagaacat tgttcgctgc attggggtga gcctgcaatc cctgccccgg 960
ttcatcctgc tggagctcat ggcgggggga gacctcaagt 1000

<210> 4
<211> 15
<212> DNA
<213> Artificial Sequence

<220>
<223> Read Seq 1 - Example sequence

<400> 4
gtaaggcacg tcata 15

<210> 5
<211> 23
<212> DNA

<213> Artificial Sequence

<220>
<223> Reference Sequence

<400> 5
gccatggaca gaaggcgcag ggc 23

<210> 6
<211> 13
<212> DNA
<213> Artificial Sequence

<220>
<223> Read Sequence R1

<400> 6
gccatggaca gaa 13

<210> 7
<211> 13
<212> DNA
<213> Artificial Sequence

<220>
<223> Read Sequence R2

<400> 7
gccatgcaca gaa 13

<210> 8
<211> 15
<212> DNA
<213> Artificial Sequence

<220>
<223> Read Sequence R3

<220>
<221> misc_feature
<222> 11..12
<223> /notc="In comparison to reference sequence SEQ ID NO 5, 2 nucleotides (with sequence "aa") corresponding to positions 12 and 13 of sequence SEQ ID NO 5, havc bccn deleted."

<400> 8
gccatggaca gggcg 15

<210> 9
<211> 21
<212> DNA
<213> Artificial Sequence

<220>
<223> Read Sequence R4

<220>
<221> misc_feature
<222> 12..15
<223> /note="Insertion of 4 nucleotides (cggt) in comparison to reference sequence SEQ ID NO 5"

<400> 9

gccatggaca gcggtaaggc g 21

The present invention includes, inter alia, the following items.

1. A quality evaluation method performed in a genetic test for testing a gene in a sample collected from a subject, for a plurality of types of gene mutations that include a first type gene mutation and a second type gene mutation different from the first type gene mutation, the quality evaluation method comprising:

preparing a quality control sample that includes a first reference gene having the first type gene mutation, and a second reference gene having the second type gene mutation;
obtaining sequence information of the genes included in the quality control sample; and
outputting an index for evaluation of a quality of the genetic test, based on the sequence information having been obtained.

2. The quality evaluation method of item 1, wherein

the first type gene mutation is substitution, deletion, or insertion of nucleotide, polymorphism, gene copy number abnormalities, or gene fusion, and
the second type gene mutation is substitution, deletion, or insertion of nucleotide, polymorphism, gene copy number abnormalities, or gene fusion, which is different from the first type gene mutation.

3. The quality evaluation method of item 1 or 2, wherein the quality control sample includes a reference gene having at least one gene mutation for each of a plurality of mutation types to be detected in the genetic test.

4. The quality evaluation method of any one of items 1 to 3, wherein a quality of the genetic test is evaluated based on the index.

5. The quality evaluation method of any one of items 1 to 4, wherein both sequence information of a gene included in the quality control sample and sequence information of a gene included in a sample are obtained.

6. The quality evaluation method of any one of items 1 to 5, wherein the quality control sample further includes a gene that does not have mutation.

7. The quality evaluation method of any one of items 1 to 6, wherein a quality of the genetic test is evaluated based on whether or not the index satisfies a predetermined criterion.

8. The quality evaluation method of item 7, wherein the predetermined criterion is different between a first gene panel used in the genetic test and a second gene panel different from the first gene panel.

9. The quality evaluation method of any one of items 1 to 8, wherein

sequence information of a gene included in the quality control sample is obtained by a sequencer, and
the index represents a quality of reading of the sequence information by the sequencer.

10. The quality evaluation method of item 9, wherein the index represents an accuracy for each base in a gene sequence read by the sequencer.

11. The quality evaluation method of item 9, wherein

the sequencer amplifies genes on a flow cell to form clusters, and
the index represents a degree to which the clusters of each gene are close to each other on the flow cell.

12. The quality evaluation method of any one of items 1 to 11, wherein

sequence information of a gene included in the quality control sample is obtained by the sequencer, and

the index represents a proportion of bases in a target region to bases having been read by the sequencer.

13. The quality evaluation method of any one of items 1 to 12, wherein the index represents a depth of sequence information, of a gene included in the quality control sample, which has been obtained.

14. A quality evaluation apparatus for evaluating a quality of a genetic test for testing a gene in a sample collected from a subject, for at least a first type gene mutation and a second type gene mutation different from the first type gene mutation, the quality evaluation apparatus comprising:

a data adjustment unit configured to analyze sequence information of a gene in a quality control sample that includes a first reference gene having the first type gene mutation and a second reference gene having the second type gene mutation; and
a quality management unit configured to generate an index for evaluation of a quality of the genetic test, based on the sequence information.

15. A quality control sample for use in a genetic test for testing a gene in a sample collected from a subject, for at least a first type gene mutation and a second type gene mutation different from the first type gene mutation, the quality control sample comprising:
a first reference gene having the first type gene mutation, and a second reference gene having the second type gene mutation.

## Claims

**1.** A quality control method for evaluating the quality of a genetic sequence analysis of a specimen obtained from a subject, wherein the specimen comprises genomic DNA, the method comprising the steps of:

preparing a quality control sample comprising a plurality of reference genes,
wherein the reference genes correspond to the target genes to be analyzed in the genetic sequence analysis,
wherein the reference genes have the different mutations to be detected in the genetic sequence analysis;
optionally performing a pre-treatment of the quality control sample and the specimen under identical conditions;
performing genetic sequence analysis of the quality control sample and the specimen to obtain read sequence information of the reference genes and the corresponding target genes comprised in the genomic DNA,
wherein the read sequence information comprises at least the base sequence and the quality score of each base in the sequence,
wherein the quality score represents at least the accuracy for each base read; and
generating a quality evaluation index based on the obtained read sequence information of the quality control sample.

**2.** The method of claim 1, wherein the quality evaluation index is generated based on the obtained read sequence information of the quality control sample and of the specimen.

**3.** The method of any one of claims 1-2, further comprising the following steps:

performing alignment of the read sequence of each nucleic acid fragment which is included in the read sequence information by mapping the read sequence of each nucleic acid fragment to the reference gene sequences to obtain at least a degree of matching,
wherein the degree of matching is a value that represents a degree of matching to which the obtained read sequence information and the reference sequence match with each other;
calculating a score representing the degree of matching between the read sequence and the reference sequence;
generating a quality evaluation index based on the obtained read sequence information of the quality control sample and the specimen and the score representing the degree of matching.

**4.** The method of any one of claims 1-3, wherein the sequence information is obtained by a sequencer.

**5.** The method of any one of claims 1-4, wherein the genetic sequence analysis is performed using next-generation sequencing, optionally wherein the genetic sequence analysis is performed using deep sequencing.

**6.** The method of any one of claims 1-5, wherein the sequencing is performed using ionic semiconductor sequencing, pyrosequencing, sequencing-by-synthesis which uses a reversible dye terminator, sequencing-by-ligation, and/or sequencing by probe ligation of oligonucleotide.

**7.** The method of any one of claims 1-6, wherein

the sequencer amplifies genes on a flow cell to form clusters, and
the index represents a degree to which the clusters of each gene formed on the flow cell are close to each other when the sequencing is performed.

**8.** The method of any one of claims 1-7, wherein
the index represents a proportion of bases in a target region to bases having been read by the sequencer.

**9.** The method of claim 8, wherein the index is is calculated as a ratio between the total number of bases in the target region and the total number of bases having been read.

**10.** The method of any one of claims 1-9, wherein the index represents a depth of read sequence information, optionally wherein the depth represents the total number of pieces of the read sequence information having been read for one base.

**11.** The method of claim 10, wherein the index is calculated as a ratio between the total number of bases, among the bases having been read, having depths which are greater than or equal to a predetermined value, and the total number of bases having been read.

**12.** The method of any one of claims 1-11, wherein the index represents a variation in depth of the read sequence information.

**13.** The method of claim 12, wherein the index is an index representing the uniformity of the depth, optionally wherein the uniformity is represented as numbers by using the interquartile range (IQR).

**14.** The method of any one of claims 1-13, wherein the index is an index indicating whether or not all the mutations in each reference gene included in the quality control sample have been detected.

**15.** The method of any one of claims 1-14, wherein the wherein the quality score (Q) is represented by the following equation:

$$Q=-10\log_{10}E$$

wherein E represents an estimated value of the probability of incorrect base assignment.

FIG. 1

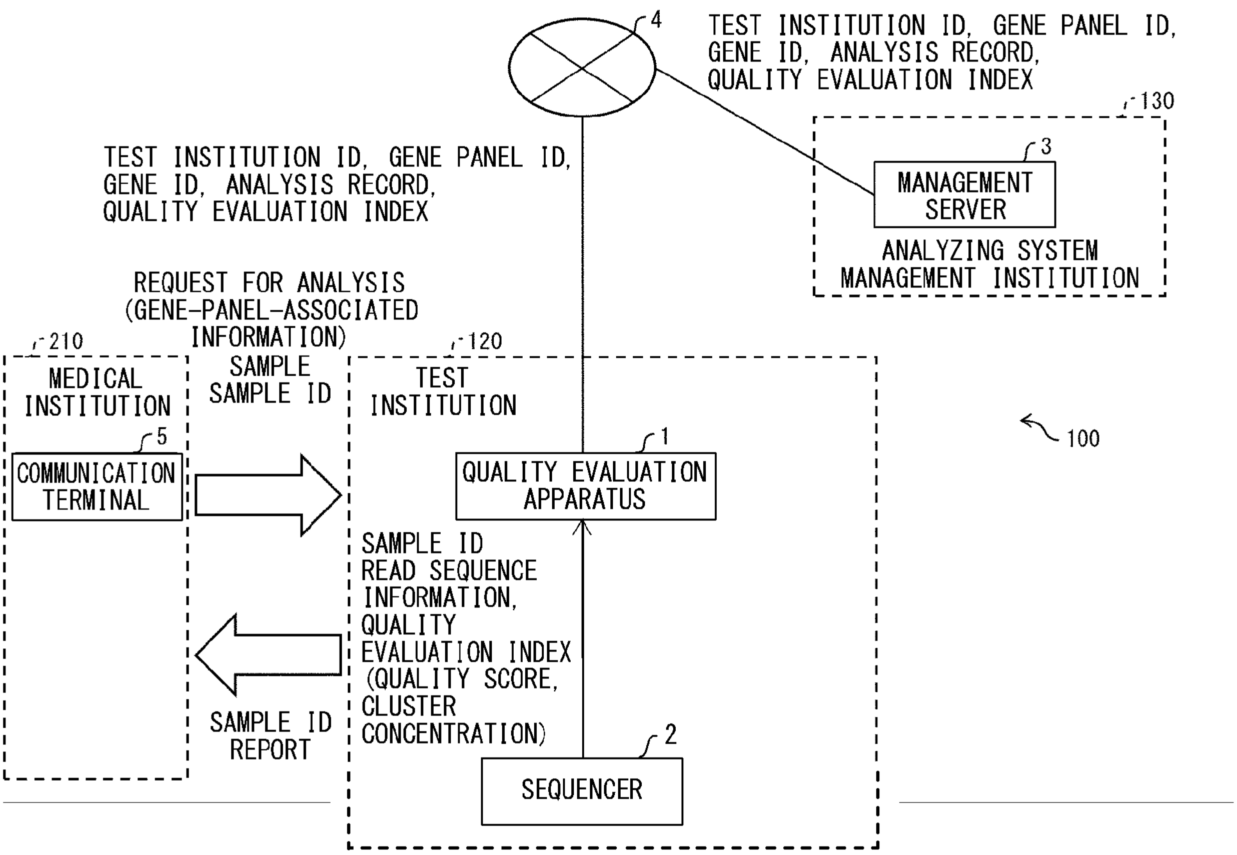
4
TEST INSTITUTION ID, GENE PANEL ID,
GENE ID, ANALYSIS RECORD,
QUALITY EVALUATION INDEX
130
3
MANAGEMENT SERVER
ANALYZING SYSTEM MANAGEMENT INSTITUTION
TEST INSTITUTION ID, GENE PANEL ID,
GENE ID, ANALYSIS RECORD,
QUALITY EVALUATION INDEX
REQUEST FOR ANALYSIS
(GENE-PANEL-ASSOCIATED INFORMATION)
SAMPLE
SAMPLE ID
210
MEDICAL INSTITUTION
5
COMMUNICATION TERMINAL
120
TEST INSTITUTION
1
QUALITY EVALUATION APPARATUS
100
SAMPLE ID
READ SEQUENCE INFORMATION,
QUALITY EVALUATION INDEX
(QUALITY SCORE,
CLUSTER CONCENTRATION)
SAMPLE ID
REPORT
2
SEQUENCER

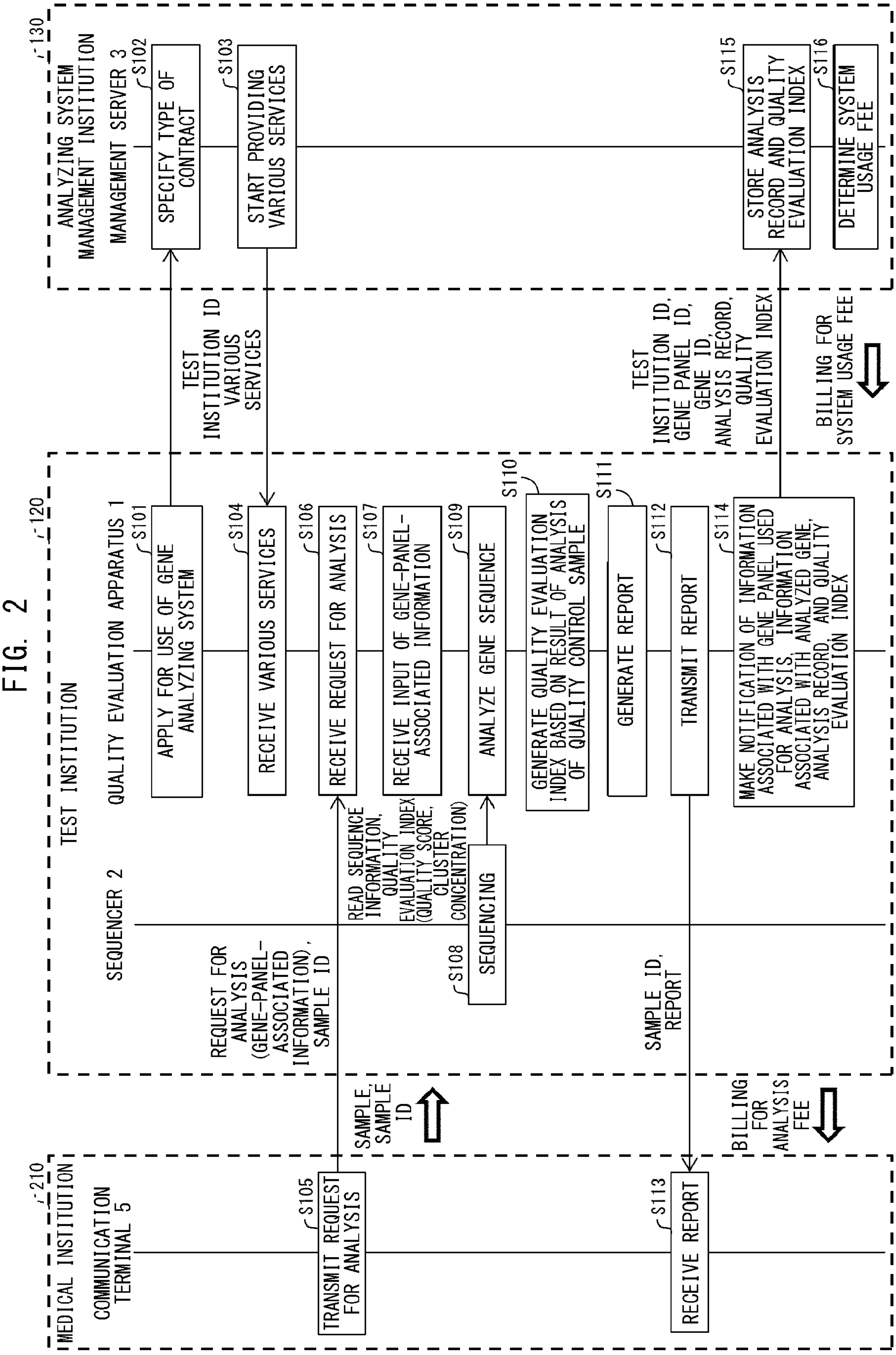

130
ANALYZING SYSTEM MANAGEMENT INSTITUTION
MANAGEMENT SERVER 3
S102 SPECIFY TYPE OF CONTRACT
S103 START PROVIDING VARIOUS SERVICES
S115 STORE ANALYSIS RECORD AND QUALITY EVALUATION INDEX
S116 DETERMINE SYSTEM USAGE FEE
TEST INSTITUTION ID
VARIOUS SERVICES
TEST INSTITUTION ID, GENE PANEL ID, GENE ID, ANALYSIS RECORD, QUALITY EVALUATION INDEX
BILLING FOR SYSTEM USAGE FEE
120
TEST INSTITUTION
QUALITY EVALUATION APPARATUS 1
S101 APPLY FOR USE OF GENE ANALYZING SYSTEM
S104 RECEIVE VARIOUS SERVICES
S106 RECEIVE REQUEST FOR ANALYSIS
S107 RECEIVE INPUT OF GENE-PANEL-ASSOCIATED INFORMATION
S109 ANALYZE GENE SEQUENCE
S110 GENERATE QUALITY EVALUATION INDEX BASED ON RESULT OF ANALYSIS OF QUALITY CONTROL SAMPLE
S111 GENERATE REPORT
S112 TRANSMIT REPORT
S114 MAKE NOTIFICATION OF INFORMATION ASSOCIATED WITH GENE PANEL USED FOR ANALYSIS, INFORMATION ASSOCIATED WITH ANALYZED GENE, ANALYSIS RECORD, AND QUALITY EVALUATION INDEX
SEQUENCER 2
REQUEST FOR ANALYSIS (GENE-PANEL-ASSOCIATED INFORMATION), SAMPLE ID
READ SEQUENCE INFORMATION, QUALITY EVALUATION INDEX (QUALITY SCORE, CLUSTER CONCENTRATION)
S108 SEQUENCING
SAMPLE ID, REPORT
SAMPLE, SAMPLE ID
BILLING FOR ANALYSIS FEE
210
MEDICAL INSTITUTION
COMMUNICATION TERMINAL 5
S105 TRANSMIT REQUEST FOR ANALYSIS
S113 RECEIVE REPORT


FIG. 2

FIG. 3

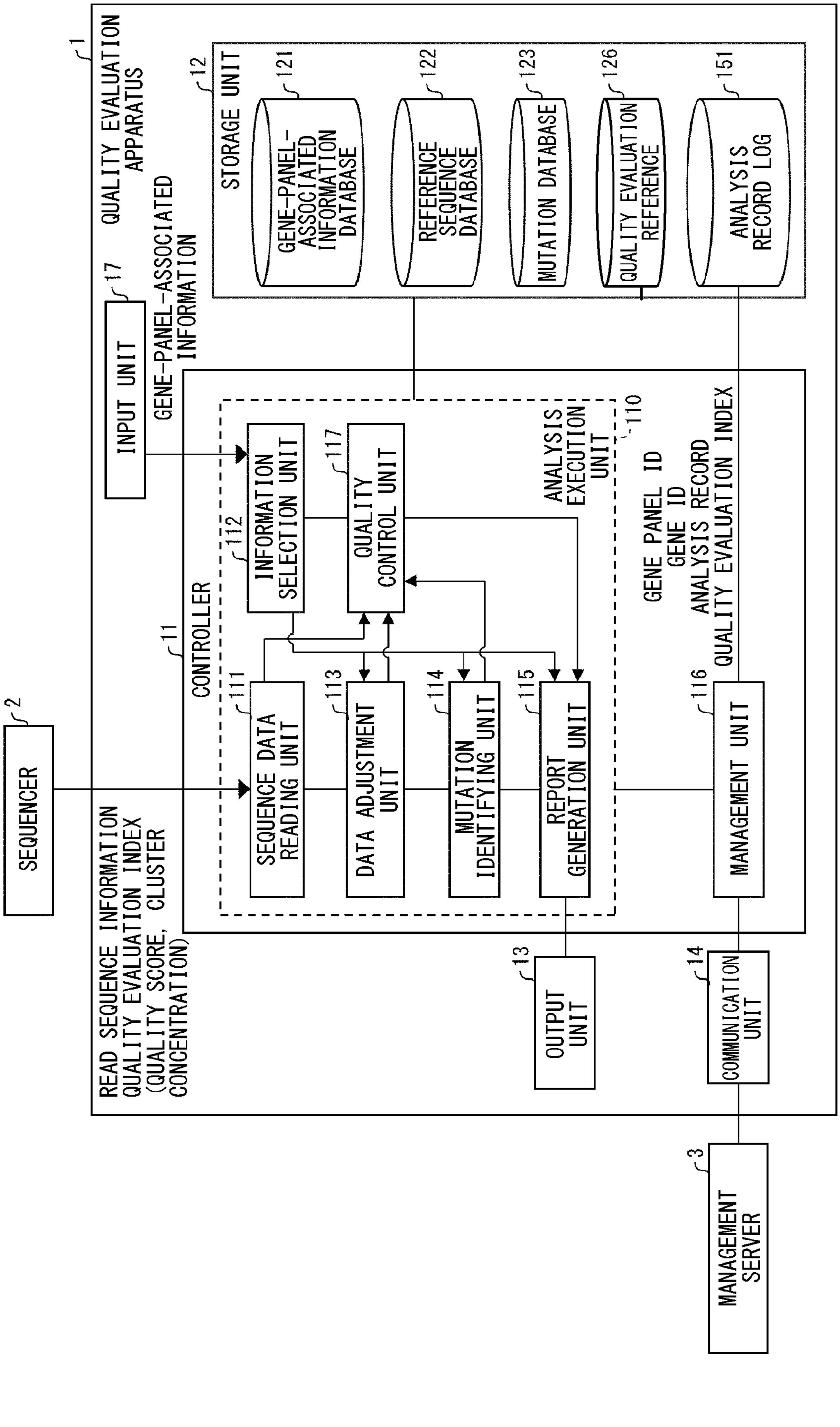
1 QUALITY EVALUATION APPARATUS
2 SEQUENCER
3 MANAGEMENT SERVER
READ SEQUENCE INFORMATION
QUALITY EVALUATION INDEX
(QUALITY SCORE, CLUSTER CONCENTRATION)
17 INPUT UNIT
GENE-PANEL-ASSOCIATED INFORMATION
11 CONTROLLER
110 ANALYSIS EXECUTION UNIT
111 SEQUENCE DATA READING UNIT
112 INFORMATION SELECTION UNIT
113 DATA ADJUSTMENT UNIT
114 MUTATION IDENTIFYING UNIT
115 REPORT GENERATION UNIT
116 MANAGEMENT UNIT
117 QUALITY CONTROL UNIT
GENE PANEL ID
GENE ID
ANALYSIS RECORD
QUALITY EVALUATION INDEX
12 STORAGE UNIT
121 GENE-PANEL-ASSOCIATED INFORMATION DATABASE
122 REFERENCE SEQUENCE DATABASE
123 MUTATION DATABASE
126 QUALITY EVALUATION REFERENCE
151 ANALYSIS RECORD LOG
13 OUTPUT UNIT
14 COMMUNICATION UNIT

FIG. 4

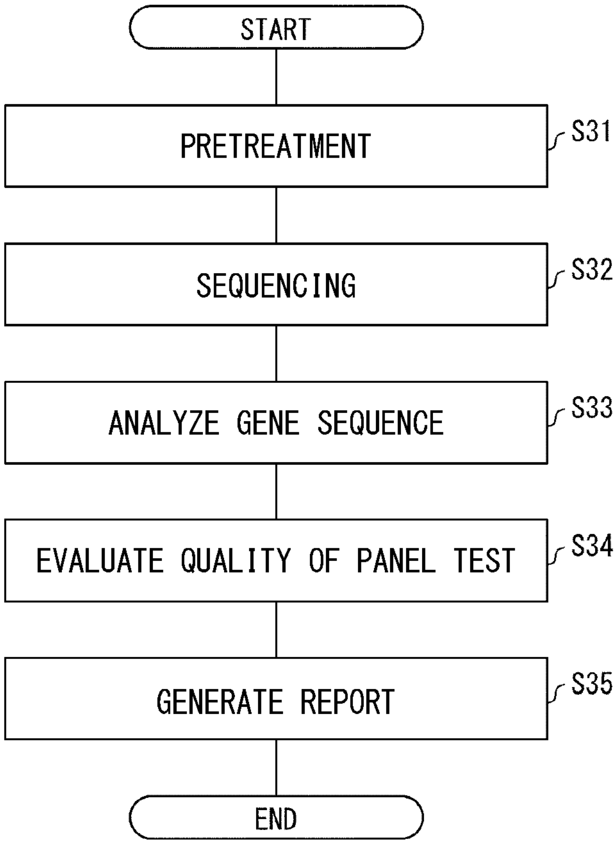
START
PRETREATMENT
S31
SEQUENCING
S32
ANALYZE GENE SEQUENCE
S33
EVALUATE QUALITY OF PANEL TEST
S34
GENERATE REPORT
S35
END

FIG. 5A

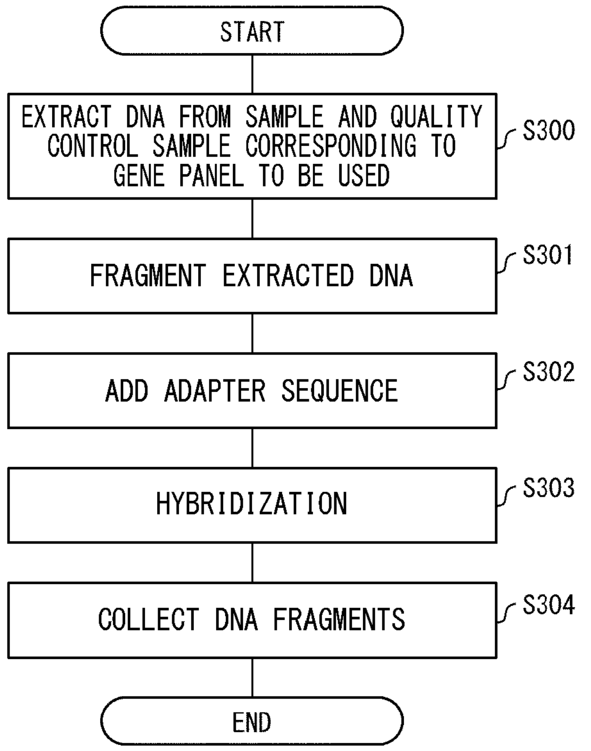


FIG. 5B

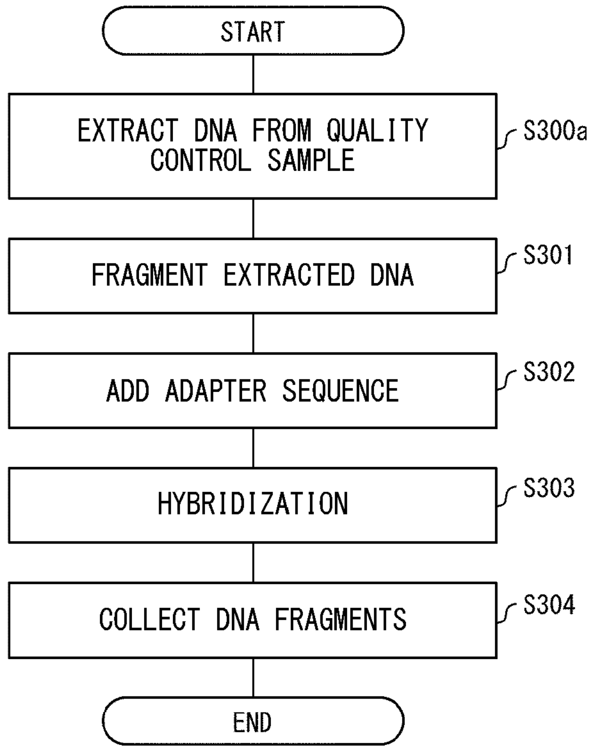


FIG. 5C

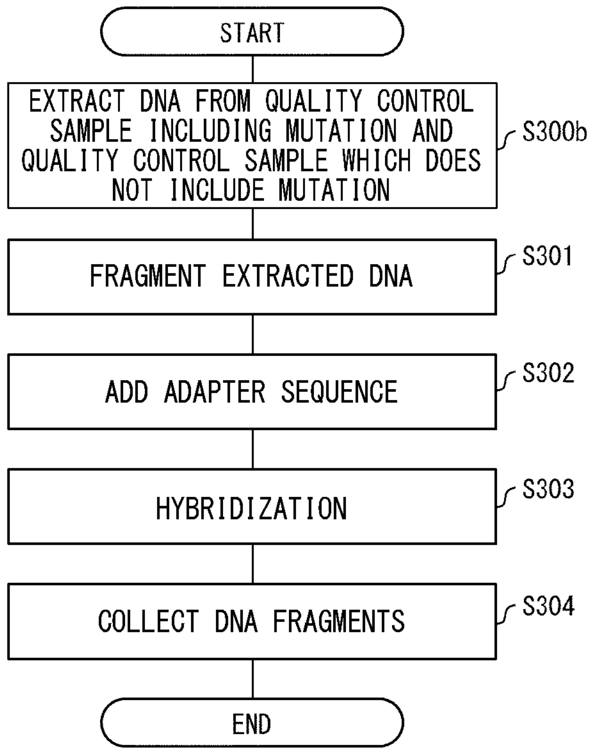


FIG. 5D

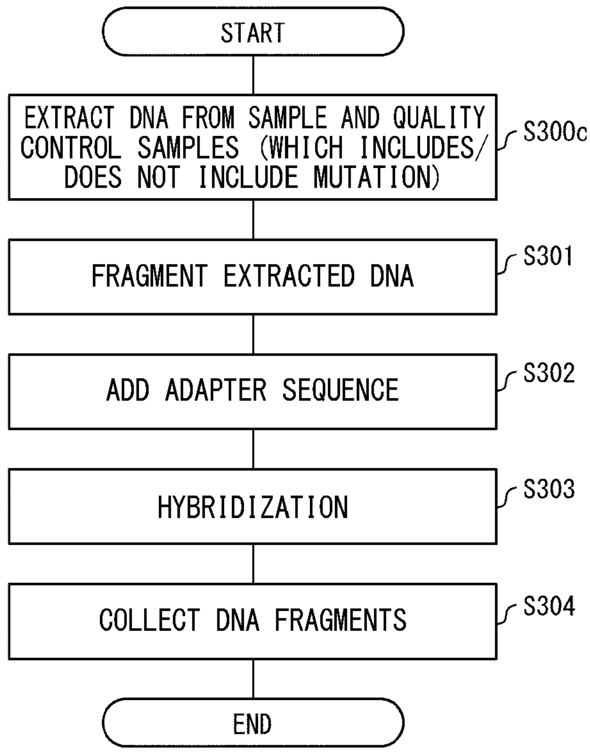

FIG. 6A

| | | | | |
|---|---|---|---|---|
| PIK3CA | EGFR | IDH1 | ROS1 | ・・・ |
| ESR1 | KRAS | IDH2 | ALK | ・・・ |
| BRAF | PDGFRA | cKIT | AKT1 | ・・・ |
| MEK | RET | ・・・ | ・・・ | ・・・ |
| S492R | ・・・ | ・・・ | ・・・ | ・・・ |
| ・・・ | ・・・ | ・・・ | ・・・ | ・・・ |
| ・・・ | ・・・ | ・・・ | ・・・ | ・・・ |

FIG. 6B

| Gene Variant Type in profiling | | | |
|---|---|---|---|
| SNV | Indel | CNV | Fusion |

FIG. 6C

| Mutation Type | Gene | Mutation |
|---|---|---|
| SNV | AAA | aaa |
| InDel | BBB | bbb |
| CNV | CCC | ccc |
| Fusion | DDD | ddd |

FIG. 6D

QUALITY CONTROL SAMPLE A

| Variant Type | Chromosome Number | Gene | Variant | Expected Allele Freq. |
|---|---|---|---|---|
| Verified Mutations | | | | |
| SNV High GC | chr.19 | GNA11 | Q209L | 5.6 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.7 | EGFR | ΔE746-A750 | 5.3 |
| Fusion | chr.4:chr.6 | ROS1 | SLC34A2/ROS1 fusion | 5.6 |
| Fusion | chr.10 | RET | CCDC6/RET fusion | 5.0 |
| CNV | chr.7 | MET | amplification | 4.5 copies |
| CNV | chr.2 | MYC-N | amplification | 9.5 copies |
| CNV | chr.8 | MYC-C | amplification | 9.8 copies |

FIG. 7

121

121B

| GENE PANEL NAME | GENE PANEL ID | ASSOCIATED GENE ID |
|---|---|---|
| ・・・ | ・・・ | ・・・ |
| A Panel | AAA | aaa, bbb, ccc, ・・・ |
| B Panel | BBB | aaa, bbb, ccc, ddd, ・・・ |
| C Panel | CCC | aaa, ccc, ・・・ |
| ・・・ | ・・・ | ・・・ |

121D

| GENE PANEL | QUALITY CONTROL SAMPLE WHICH INCLUDES MUTATION | QUALITY CONTROL SAMPLE WHICH DOES NOT INCLUDE MUTATION |
|---|---|---|
| A Panel | REFERENCE SAMPLE A1<br>REFERENCE SAMPLE A2<br>⋮ | REFERENCE SAMPLE M1<br>REFERENCE SAMPLE M2<br>⋮ |
| B Panel | REFERENCE SAMPLE B1<br>REFERENCE SAMPLE B2<br>⋮ | REFERENCE SAMPLE N1<br>REFERENCE SAMPLE N2<br>⋮ |

FIG. 8A

QUALITY CONTROL SAMPLE A

| Variant Type | Chromosome Number | Gene | Variant | Expected Allele Freq. |
|---|---|---|---|---|
| Verified Mutations | | | | |
| SNV High GC | chr.19 | GNA11 | Q209L | 5.6 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.7 | EGFR | ΔE746-A750 | 5.3 |
| Fusion | chr.4:chr.6 | ROS1 | SLC34A2/ROS1 fusion | 5.6 |
| Fusion | chr.10 | RET | CCDC6/RET fusion | 5.0 |
| CNV | chr.7 | MET | amplification | 4.5 copies |
| CNV | chr.2 | MYC-N | amplification | 9.5 copies |
| CNV | chr.8 | MYC-C | amplification | 9.8 copies |

a1 a2 a3

FIG. 8B

QUALITY CONTROL SAMPLE B

| Variant Type | Chromosome Number | Gene | Variant | Expected Allele Freq. |
|---|---|---|---|---|
| Verified Mutations | | | | |
| SNV High GC | chr.19 | GNA11 | R183C | 5.9 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.16 | TSC2 | ΔH1746-R1751 | 5.5 |
| Fusion | chr.4:chr.6 | ROS1 | SLC34A2/ROS1 fusion | 5.6 |
| Fusion | chr.10 | RET | CCDC6/RET fusion | 5.0 |
| Fusion | chr.9 | ABL1 | BCR/ABL1 fusion | 5.1 copies |
| CNV | chr.2 | MYC-N | amplification | 9.5 copies |
| CNV | chr.8 | MYC-C | amplification | 9.8 copies |

b1 b2 b3

FIG. 9A FRAGMENTATION OF DNA

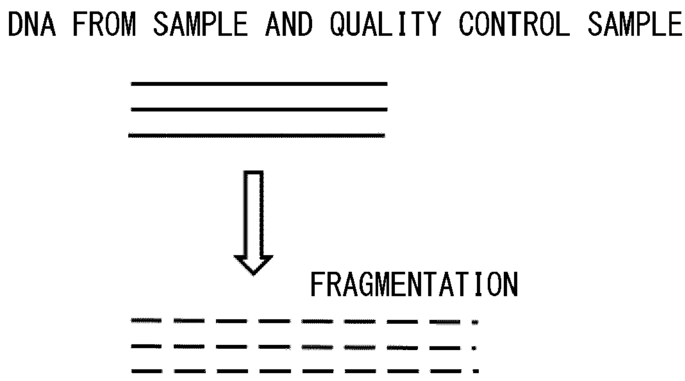


FIG. 9B ADD ADAPTER SEQUENCE

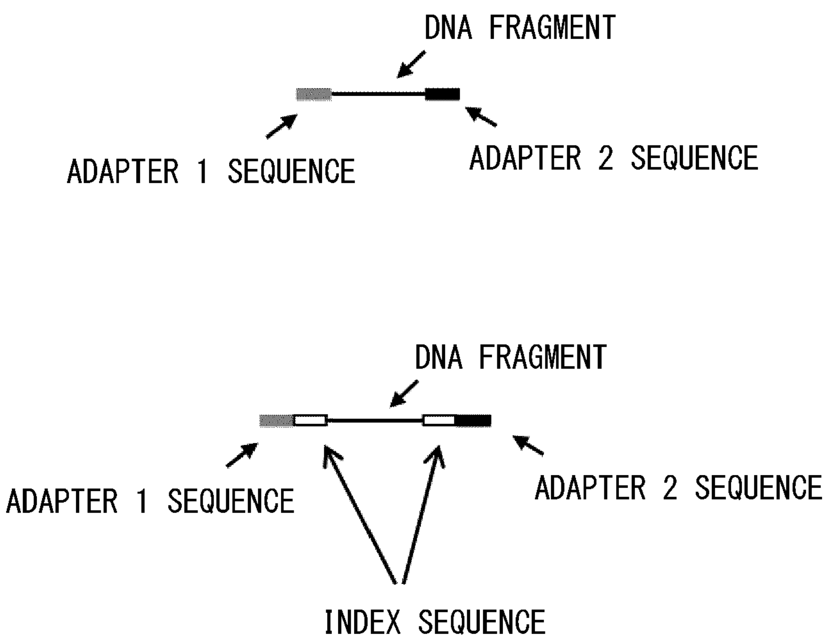

FIG. 10

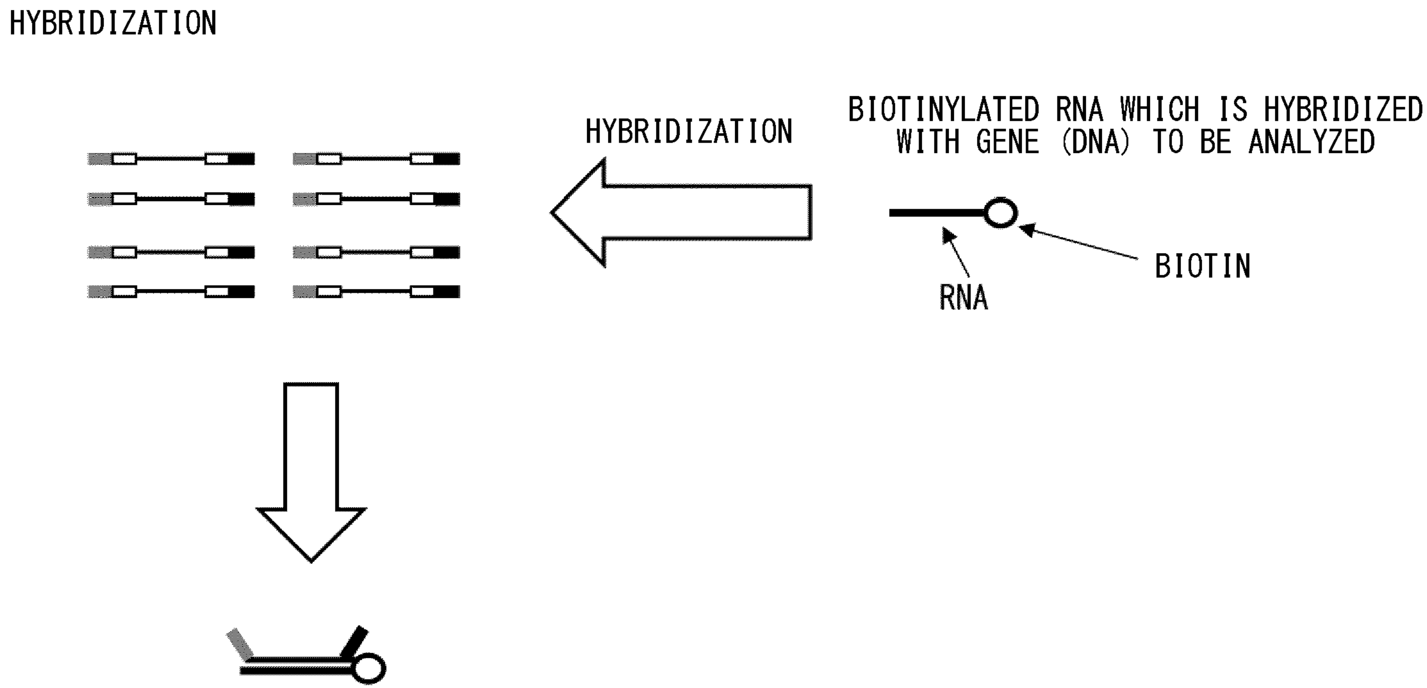
HYBRIDIZATION
HYBRIDIZATION
BIOTINYLATED RNA WHICH IS HYBRIDIZED
WITH GENE (DNA) TO BE ANALYZED
BIOTIN
RNA

FIG. 11

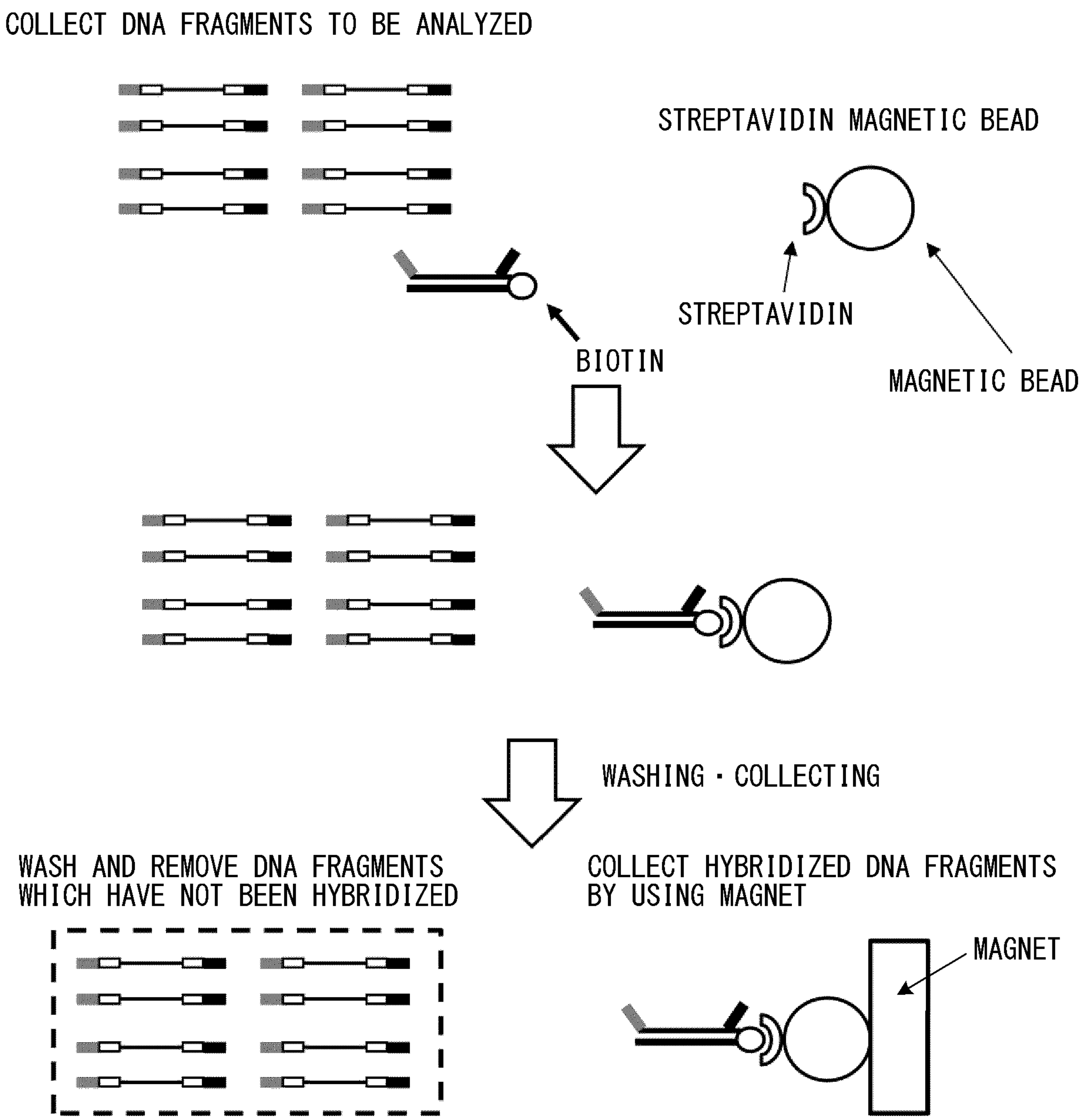
COLLECT DNA FRAGMENTS TO BE ANALYZED
STREPTAVIDIN MAGNETIC BEAD
STREPTAVIDIN
BIOTIN
MAGNETIC BEAD
WASHING・COLLECTING
WASH AND REMOVE DNA FRAGMENTS
WHICH HAVE NOT BEEN HYBRIDIZED
COLLECT HYBRIDIZED DNA FRAGMENTS
BY USING MAGNET
MAGNET

FIG. 12

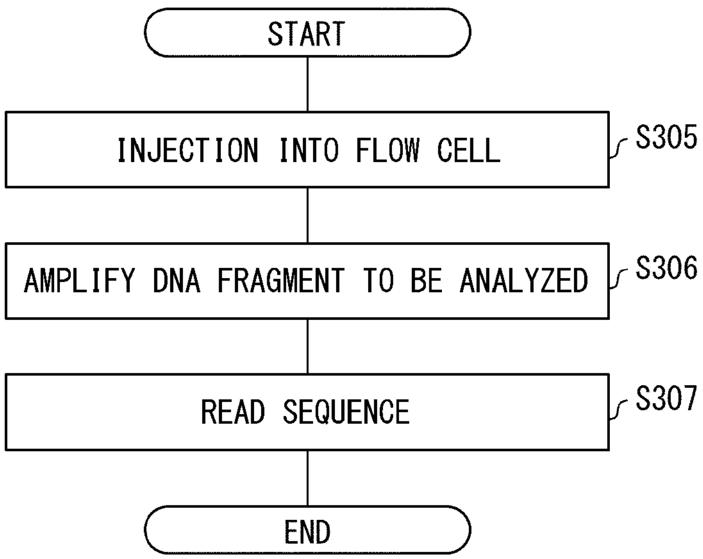
START
INJECTION INTO FLOW CELL
S305
AMPLIFY DNA FRAGMENT TO BE ANALYZED
S306
READ SEQUENCE
S307
END

FIG. 13

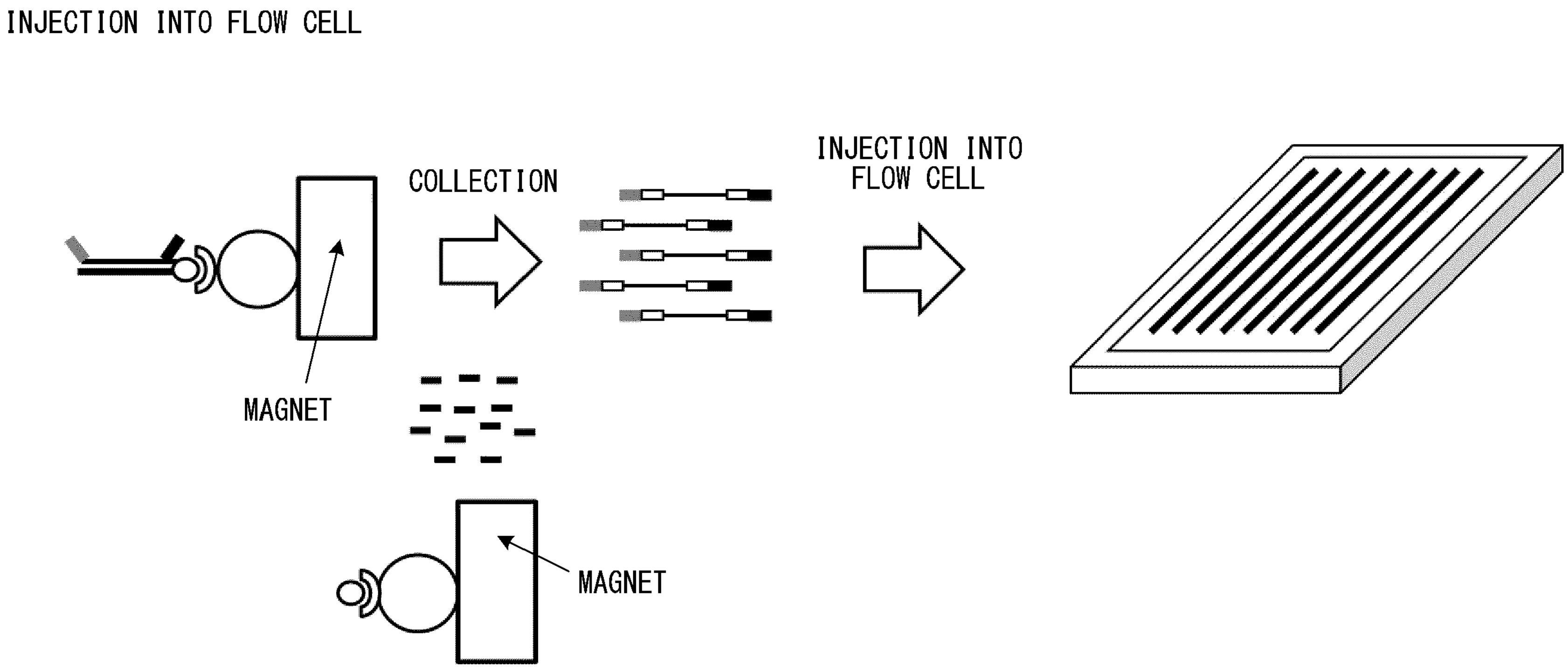
INJECTION INTO FLOW CELL
COLLECTION
INJECTION INTO FLOW CELL
MAGNET
MAGNET

FIG. 14

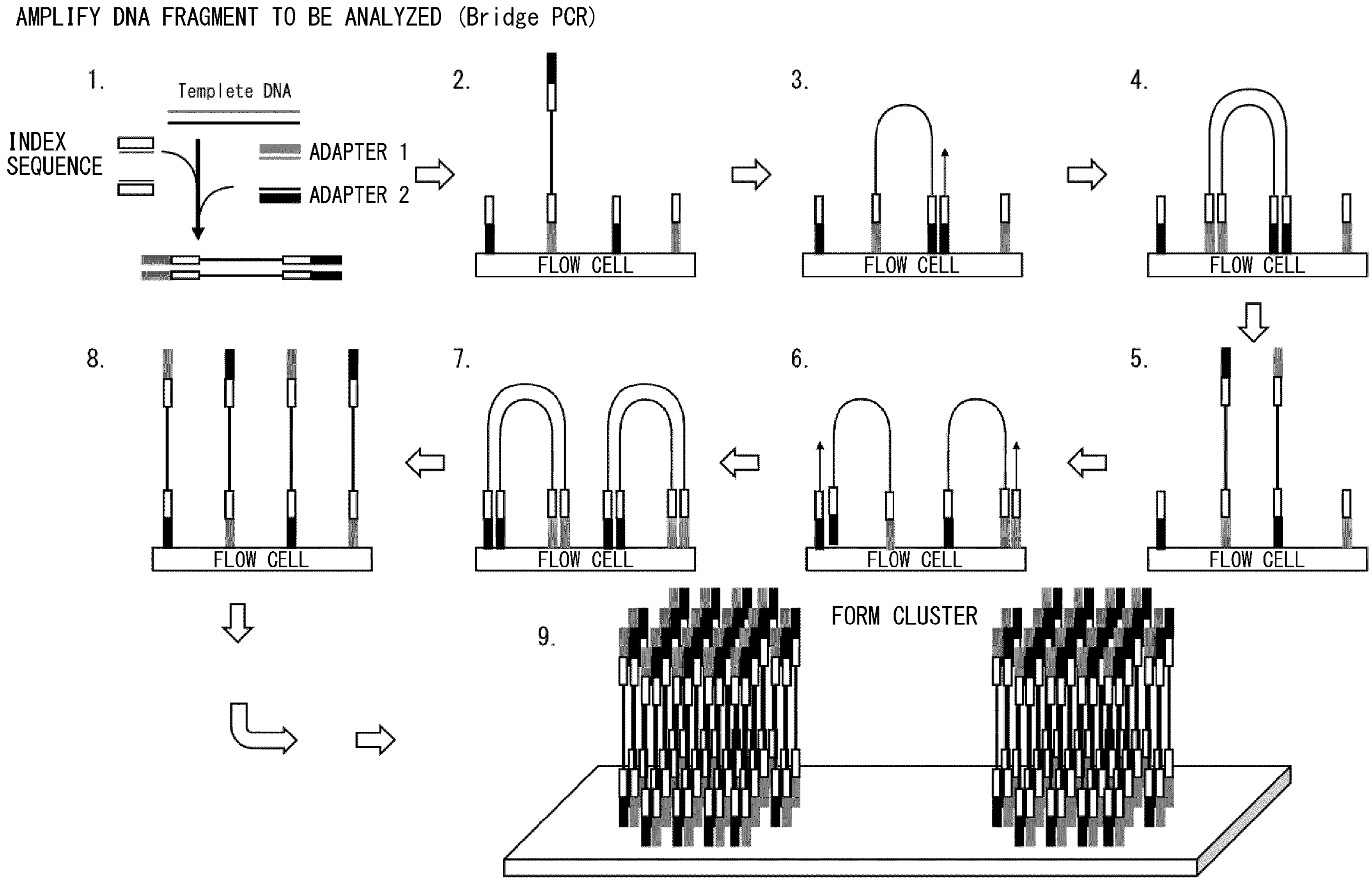
AMPLIFY DNA FRAGMENT TO BE ANALYZED (Bridge PCR)
1.
Templete DNA
INDEX SEQUENCE
ADAPTER 1
ADAPTER 2
2.
FLOW CELL
3.
FLOW CELL
4.
FLOW CELL
5.
FLOW CELL
6.
FLOW CELL
7.
FLOW CELL
8.
FLOW CELL
9.
FORM CLUSTER

FIG. 15

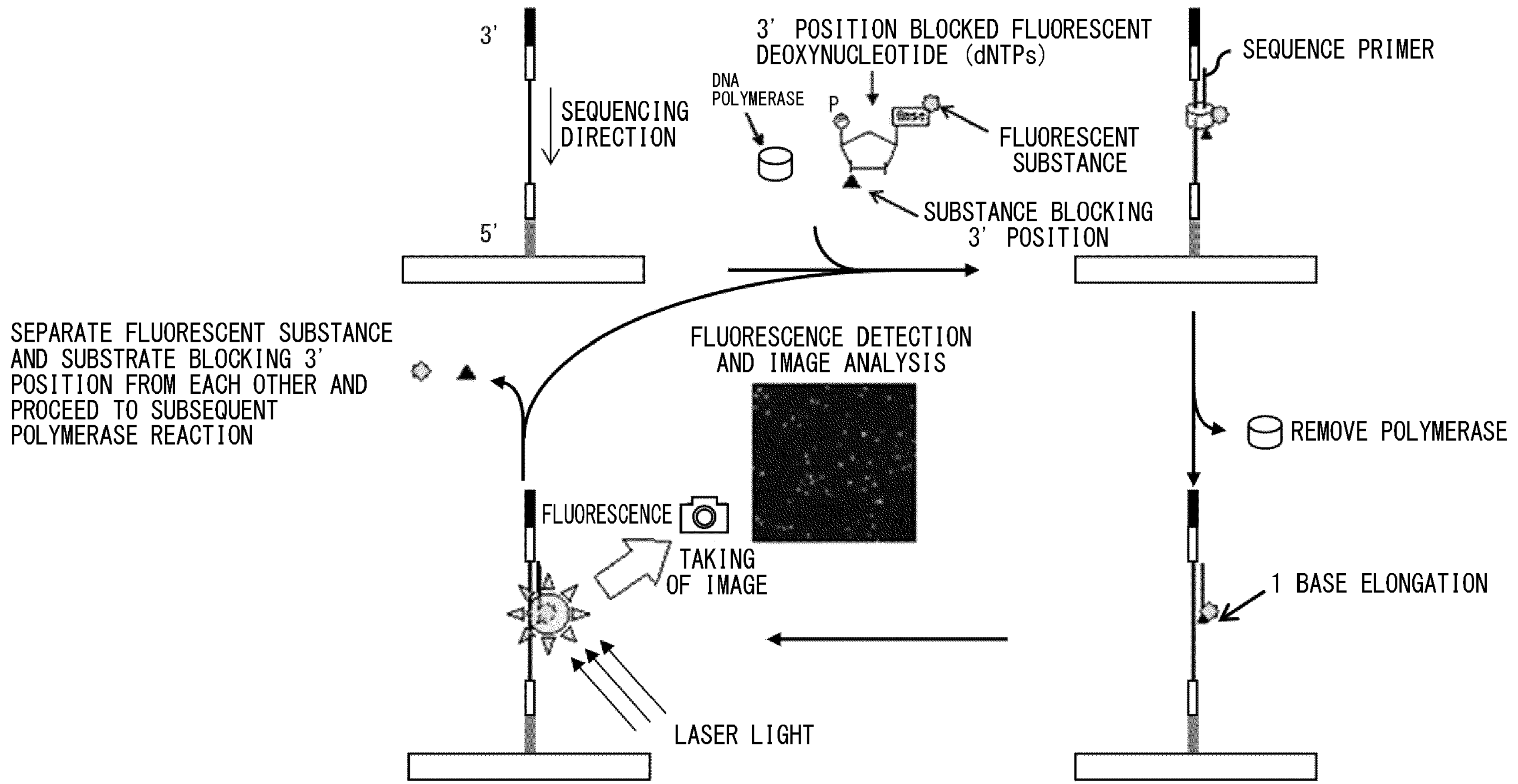
3'
5'
SEQUENCING DIRECTION
DNA POLYMERASE
3' POSITION BLOCKED FLUORESCENT DEOXYNUCLEOTIDE (dNTPs)
P
FLUORESCENT SUBSTANCE
SUBSTANCE BLOCKING 3' POSITION
SEQUENCE PRIMER
REMOVE POLYMERASE
1 BASE ELONGATION
FLUORESCENCE DETECTION AND IMAGE ANALYSIS
FLUORESCENCE
TAKING OF IMAGE
LASER LIGHT
SEPARATE FLUORESCENT SUBSTANCE AND SUBSTRATE BLOCKING 3' POSITION FROM EACH OTHER AND PROCEED TO SUBSEQUENT POLYMERASE REACTION

FIG. 16

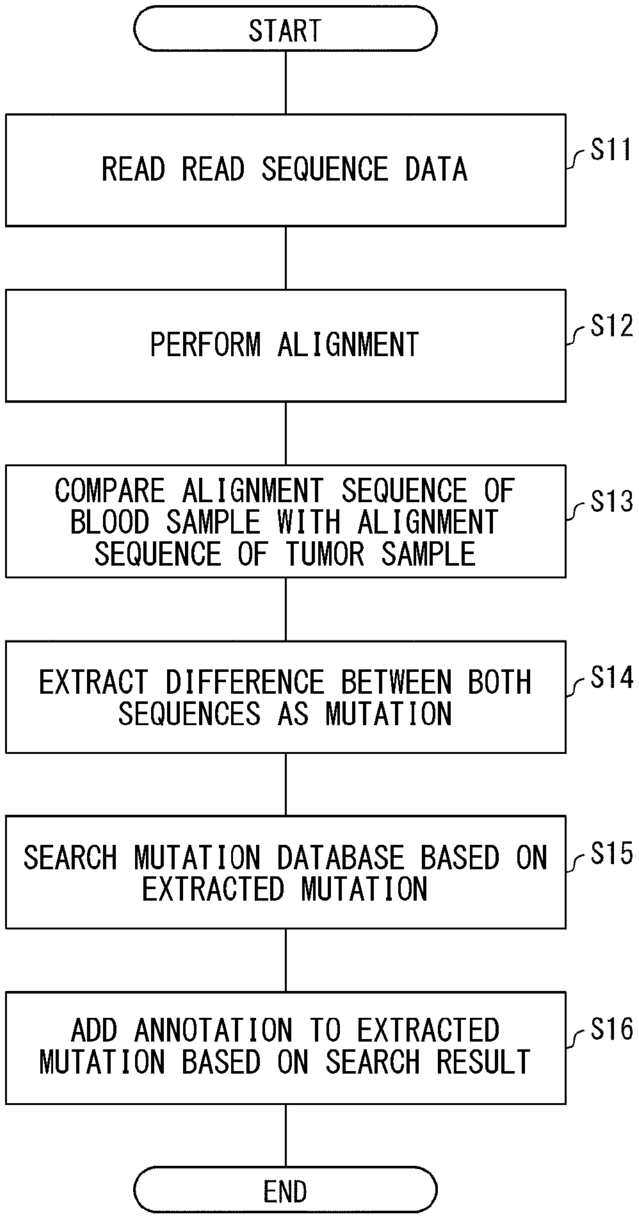
START
READ READ SEQUENCE DATA
S11
PERFORM ALIGNMENT
S12
COMPARE ALIGNMENT SEQUENCE OF BLOOD SAMPLE WITH ALIGNMENT SEQUENCE OF TUMOR SAMPLE
S13
EXTRACT DIFFERENCE BETWEEN BOTH SEQUENCES AS MUTATION
S14
SEARCH MUTATION DATABASE BASED ON EXTRACTED MUTATION
S15
ADD ANNOTATION TO EXTRACTED MUTATION BASED ON SEARCH RESULT
S16
END

FIG. 17

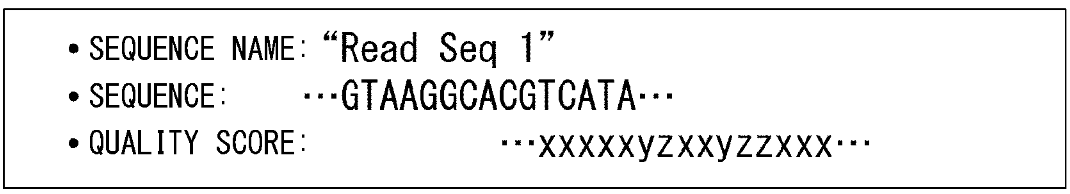
• SEQUENCE NAME: "Read Seq 1"
• SEQUENCE: ···GTAAGGCACGTCATA···
• QUALITY SCORE: ···xxxxxyzxxyzzxxx···

FIG. 18A

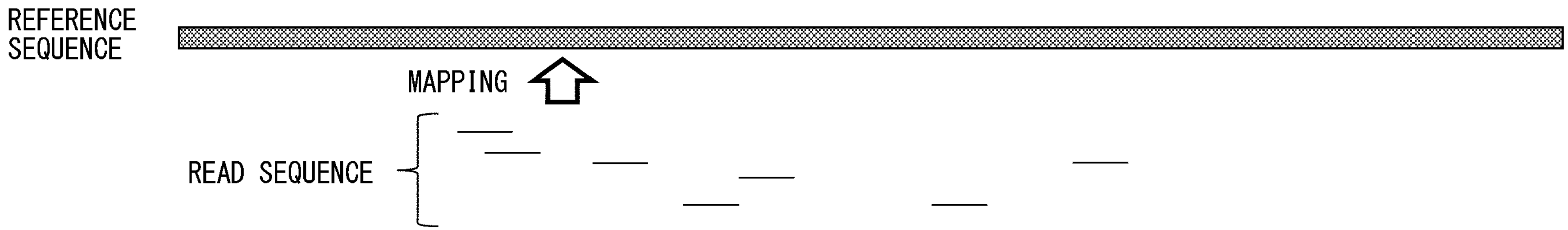


FIG. 18B

- REFERENCE SEQUENCE INFORMATION: REFERENCE SEQUENCE NAME (REFERENCE SEQUENCE ID), SEQUENCE LENGTH OF REFERENCE SEQUENCE, AND THE LIKE
- READ SEQUENCE NAME: NAME (READ SEQUENCE ID) OF EACH READ SEQUENCE FOR WHICH ALIGNMENT HAS BEEN PERFORMED
- POSITION INFORMATION: LEFTMOST MAPPING POSITION.
  POSITION ON REFERENCE SEQUENCE AT WHICH LEFTMOST BASE OF READ SEQUENCE HAS BEEN MAPPED
- MAPPING QUALITY: MAPPING QUALITY CORRESPONDING TO THE READ SEQUENCE
- SEQUENCE: BASE SEQUENCE CORRESPONDING TO EACH READ SEQUENCE (EXAMPLE: ...GTAAGGCACGTCATA...)

FIG. 19

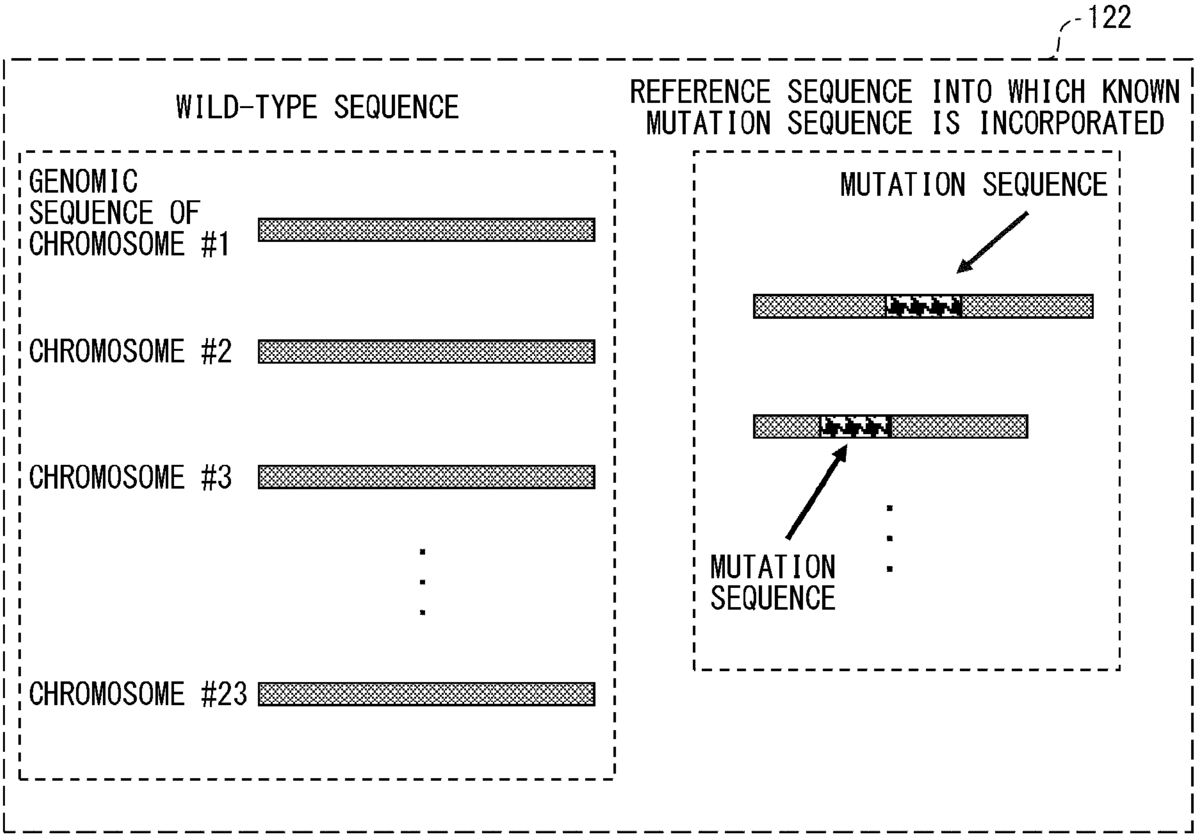


FIG. 20

| GENE NAME | POSITION OF CHROMOSOME | MUTATION |
|---|---|---|
| AKT1 | 14q32.33 | p.E17K |
| BRAF | 7q34 | p.V600E, p.V600K, p.V600, p.V600R, p.K601E, p.D594G, p.G469A, p.D594N, p.V600M, p.V600_K601>E, p.G466V, p.V600D, p.L597R, p.G469V, p.N581S, p.V600L, p.G469R, p.G469E, p.G466E, p.L597Q, p.L597S, p.V600A |
| EGFR | 7p11.2 | p.L858R, p.E746_A750del, p.E746_A750delELREA, p.T790M, p.L861Q, p.G719, p.S768I, p.L747_P753>S, p.G719A, p.L747_T751del, p.L747_A750>P, p.G719S, p.L747_T751delLREAT, p.E746_S752>V, c.3633+294delA, p.V769_D770insASV, p.L747_S752del, p.G719C, p.L747_S752delLREATS, p.G598V, p.A289V, c.3633+293_3633+294delAA, p.L747_P753del, p.D770_N771insSVD, p.E746_T751del, p.L747_T751>P, p.E709K, p.E746_T751>A, p.L833V, p.N158N, p.K745_A750del, p.H773_V774insNPH, p.C797S |
| ... | ... | ... |

FIG. 21

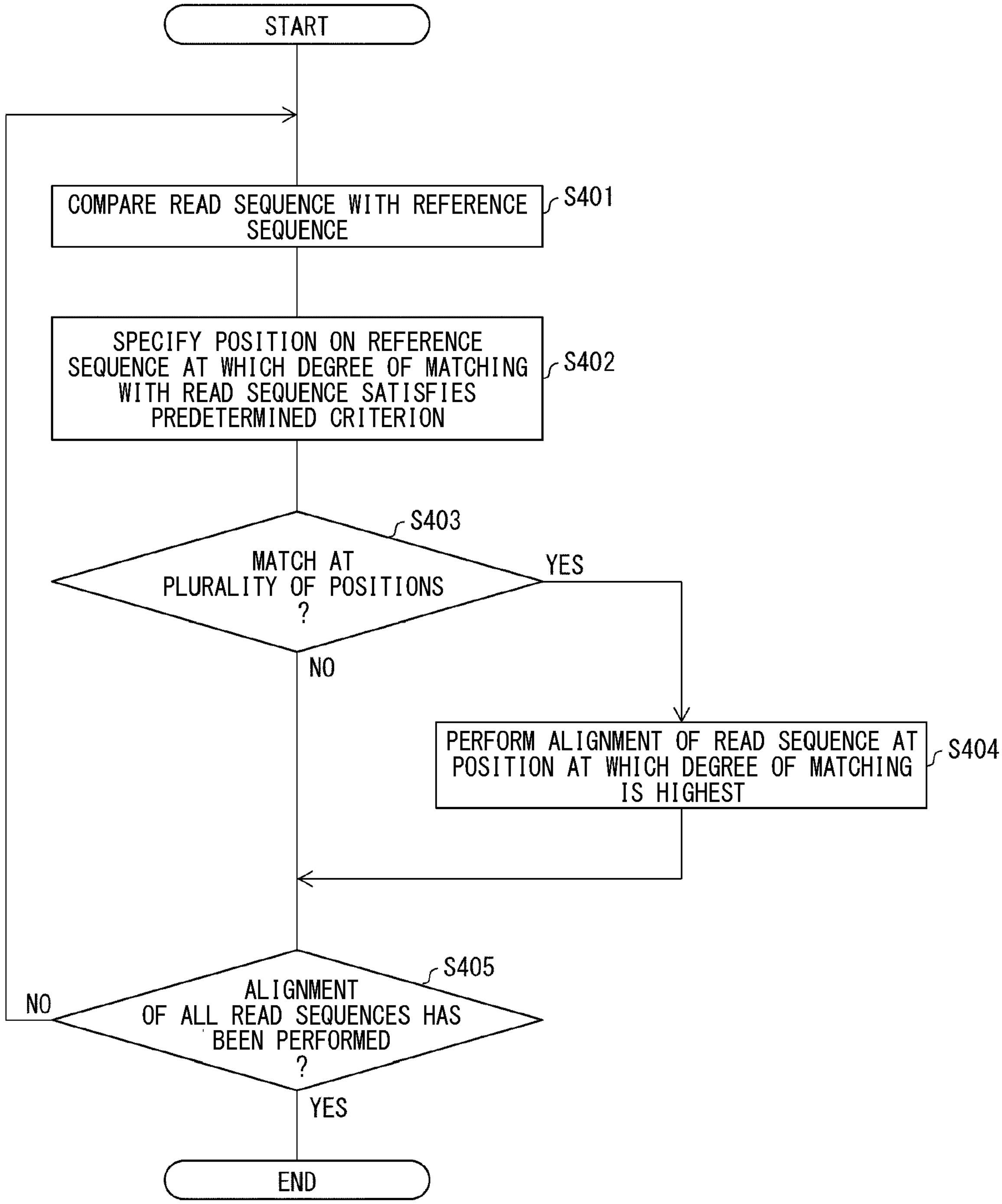
START
COMPARE READ SEQUENCE WITH REFERENCE SEQUENCE
S401
SPECIFY POSITION ON REFERENCE SEQUENCE AT WHICH DEGREE OF MATCHING WITH READ SEQUENCE SATISFIES PREDETERMINED CRITERION
S402
S403
MATCH AT PLURALITY OF POSITIONS ?
YES
NO
PERFORM ALIGNMENT OF READ SEQUENCE AT POSITION AT WHICH DEGREE OF MATCHING IS HIGHEST
S404
S405
ALIGNMENT OF ALL READ SEQUENCES HAS BEEN PERFORMED ?
NO
YES
END

FIG. 22A

REFERENCE SEQUENCE: ・・・GCCATGGACAGAAGGCGCAGGGC・・・
READ SEQUENCE R1: GCCATGGACAGAA
READ SEQUENCE R2: GCCATGCACAGAA

FIG. 22B

REFERENCE SEQUENCE: ・・・GCCATGGACAGAAGGCGCAGGGC・・・
READ SEQUENCE R3: GCCATGGACAG**GGCG
READ SEQUENCE R4: GCCATGGACAGAAGGCG

CGGT

FIG. 23

- POSITION INFORMATION: INFORMATION OF POSITION ON REFERENCE GENOME. FOR EXAMPLE, SPECIFIED BY CHROMOSOME NUMBER, AND POSITION ON THE CHROMOSOME
- REFERENCE BASE: REFERENCE BASE (A, T, C, G, OR THE LIKE) IN THE POSITION INFORMATION
- MUTATION BASE: BASE OF REFERENCE BASE WHICH IS PRESENT AFTER MUTTION

| CHROM | POS | REF | ALT | |
|---|---|---|---|---|
| 20 | 3 | C | G | |
| 21 | 4 | C | CTAG | ← EXAMPLE OF Insertion MUTATION |
| 19 | 10 | TCG | T | ← EXAMPLE OF Deletion MUTATION |
| 2 | 321681 | G | G]17:198982] | ← REVERSE COMPLEMENT SEQUENCE OF SEQUENCE THAT EXTENDS AT LEFT OF 198982 POSITION OF CHROMOSOME 17 IS BOUND AFTER "G" |
| 2 | 321682 | T | ]13:123456]T | ← SEQUENCE AT LEFT OF 123456 POSITION OF CHROMOSOME 13 IS BOUND BEFORE "T" |
| 13 | 123456 | C | C[2:321682[ | ← SEQUENCE AT RIGHT OF 321682 POSITION OF CHROMOSOME 2 IS BOUND AFTER "C" |
| 13 | 123457 | A | [17:198983[A | ← REVERSE COMPLEMENT SEQUENCE OF SEQUENCE THAT EXTENDS AT RIGHT OF 198983 POSITION OF CHROMOSOME 17 IS BOUND AFTER "C" |

FIG. 24

123

| MUTATION ID | CHROM | POS | REF | ALT | Annotation | METADATA ASSOCIATED WITH GENE-PANEL-ASSOCIATED INFORMATION |
|---|---|---|---|---|---|---|
| #1 | 20 | 3 | C | G | abc | GENE ID: a |
| #2 | 19 | 4 | A | T | xyz | GENE ID: b |
| #3 | xx | yy | C | G | EGFR L858R | GENE ID: c |
| #4 | aa | bb | T | A | BRAF V600E | GENE ID: d |
| . . . | | | | | | |
| #xx | abc | ABC | G | G]p] | ALK-EML4 FUSION | GENE ID: e, GENE ID: f |
| #yy | xyz | XYZ | T | ]p]T | ROS1-CD74 FUSION | GENE ID: g, GENE ID: h |
| . . . | | | | | | |

FIG. 25

- MUTATION ID: IDENTIFIER FOR IDENTIFYING MUTATION
- MUTATION POSITION INFORMATION:
    - CHROM: CHROMOSOME NUMBER
    - POS: POSITION AT CHROMOSOME NUMBER
- REF: WILD TYPE BASE
- ALT: BASE AFTER MUTATION
- Annotation: INFORMATION ASSOCIATED WITH MUTATION

FIG. 26

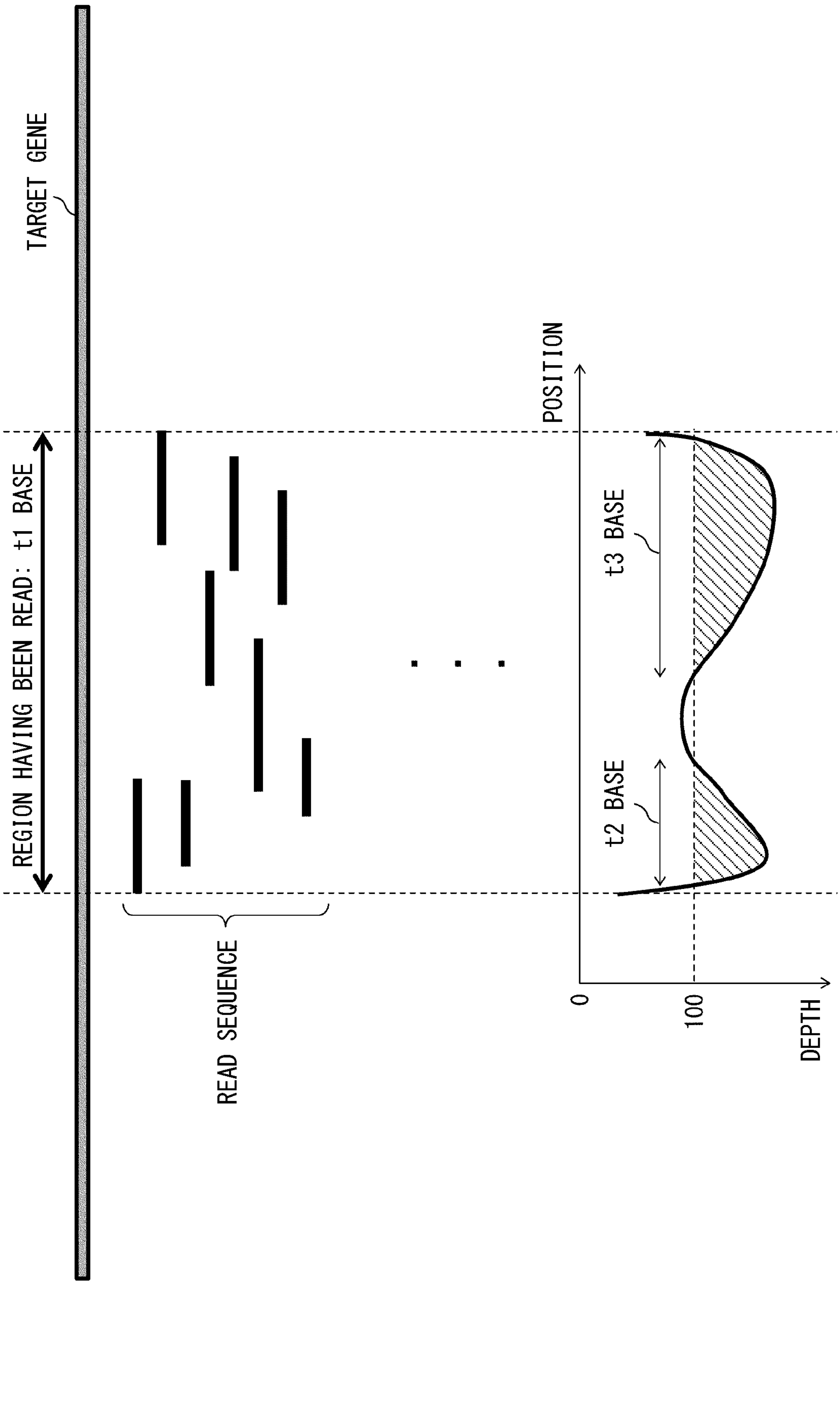
TARGET GENE
REGION HAVING BEEN READ: t1 BASE
READ SEQUENCE
POSITION
t3 BASE
t2 BASE
0
100
DEPTH

FIG. 27

REFERENCE SAMPLE A

| Variant Type | Chromosome Number | Gene | Variant | Expected Allele Freq. |
|---|---|---|---|---|
| Verified Mutations | | | | |
| SNV High GC | chr.19 | GNA11 | Q209L | 5.6 |
| SNV High GC | chr.14 | AKT1 | E17K | 5.0 |
| SNV Low GC | chr.3 | PIK3CA | E545K | 5.6 |
| Long Insertion | chr.7 | EGFR | V769_D770ins ASV | 5.6 |
| Long Deletion | chr.7 | EGFR | ΔE746-A750 | 5.3 |
| Fusion | chr.4:chr.6 | ROS1 | SLC34A2/ROS1 fusion | 5.6 |
| Fusion | chr.10 | RET | CCDC6/RET fusion | 5.0 |
| CNV | chr.7 | MET | amplification | 4.5 copies |
| CNV | chr.2 | MYC-N | amplification | 9.5 copies |
| CNV | chr.8 | MYC-C | amplification | 9.8 copies |

FIG. 28

GENE ANALYSIS RESULT REPORT

PATIENT ID: [redacted]
SEX: FEMALE
NAME OF DISEASE: xxx CANCER STAGE II
NAME OF DOCTOR IN CHARGE: [redacted]
NAME OF INSTITUTION: [redacted]
GENE PANEL: A PANEL
QC INDEX: XXXXXXX

DATE OF REQUEST: MAY 20, 2017
DATE OF MEASUREMENT: MAY 30, 2017
DATE OF REPORT: JUNE 19, 2017

DETECTED GENE MUTATION AND RELATED MEDICINE

| MARKER | CHANGE | JAPAN | | | U.S.A. | |
|---|---|---|---|---|---|---|
| | | APPROVAL | OFF LABEL | CLINICAL TRIAL | APPROVED BY FDA | OFF LABEL |
| BRAF | p.V600E | – | MEDICINE A, MEDICINE B MEDICINE C | – | – | MEDICINE A, MEDICINE B MEDICINE D MEDICINE E |
| BRCA1 | p.K1183R | – | – | MEDICINE F | MEDICINE F MEDICINE G | – |

LIST OF CLINICAL TESTS

| MARKER | TEST ID | PHASE | TARGET DISEASE | NAME OF TEST | NAME OF MEDICINE | INSTITUTION THAT HAS PERFORMED THE TEST |
|---|---|---|---|---|---|---|
| ALK; ROS1 | Jxxxxxx-xxxxxxx | Phase 1, 2 | xxx CANCER | 1/2 PHASE TEST OF MEDICINE MX FOR xxx CANCER PATIENT | MEDICINE MX | COMPANY M |
| ROS1; NTRK | Jxxxxxxx-xxxxxxx | Phase 1 | yyy CANCER | EVALUATION OF SAFETY AND PHARMACOKINETICS OF MEDICINE NY FOR yyy CANCER PATIENT | MEDICINE NY | COMPANY N |

FIG. 29

A3140G

REFERENCE GENE (PIK3CA PARTIAL SEQUENCE INCLUDING A3140G)

5' gtttcaggagatgtgttacaaggcttatctagctattcgacagcatgccaatctcttcataaatcttttc
tcaatgatgcttggctctggaatgccagaactacaatcttttgatgacattgcatacattcgaaagaccctag
ccttagataaaactgagcaagaggctttggagtatttcatgaaacaaatgaatgatgcacgtcatggtggctg
gacaacaaaaatggattggatcttccacacaattaaacagcatgcattgaactgaaagataactgagaaaatg
aaagctcactctggattccacactgcactgttaataactctcagcaggcaaagaccgattgcataggaattgc
acaatccatgaacagcattagatttacagcaagaacagaaataaaatactatataatttaaataatgtaaacg
caaacagggtttgatagcacttaaactagttcatttcaaaa 3'

FIG. 30

REFERENCE GENE (SEQUENCE OF EML4-ALK FUSION GENE)

EML4

5’ gttattgatgactccaatgagcatatgcttactgtatgggactggcagaggaaagcaaaaggagc
agaaataaagacaacaaatgaagttgttttggctgtggagtttcacccaacagatgcaaataccataa
ttacatgcggtaaatctcatattttcttctggacctggagcggcaattcactaacaagaaaacaggga
atttttgggaaatatgaaaagccaaaatttgtgcagtgtttagcattcttggggaatggagatgttct
tactggagactcaggtggagtcatgcttatatggagcaaaactactgtagagcccacacctgggaaag
gacctaaaggtgtatatcaaatcagcaaacaaatcaaagctcatgatggcagtgtgttcacactttgt
cagatgagaaatgggatgttattaactggaggagggaaagacagaaaaataattctgtgggatcatga
tctgaatcctgaaagagaaatagagtt

ALK

gagtgcttcaagggccaggctgccaggccatgttgcagct
gaccacccacctgcagtgtaccgccggaagcaccaggagctgcaagccatgcagatggagctgcagag
ccctgagtacaagctgagcaagctccgcacctcgaccatcatgaccgactacaaccccaactactgct
ttgctggcaagacctcctccatcagtgacctgaaggaggtgccgcggaaaaacatcaccctcattcgg
ggtctgggccatggcgcctttggggaggtgtatgaaggccaggtgtccggaatgcccaacgacccaag
ccccctgcaagtggctgtgaagacgctgcctgaagtgtgctctgaacaggacgaactggatttcctca
tggaagccctgatcatcagcaaattcaaccaccagaacattgttcgctgcattggggtgagcctgcaa
tccctgccccggttcatcctgctggagctcatggcgggggggagacctcaagt 3’

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017208652 A
- US 8026053 B


### Non-patent literature cited in the description


- **LIH et al.** Analytical Validation of the Next-Generation Sequencing Assay for a Nationwide Signal-Finding Clinical Trial. *The Journal of Molecular Diagnostics,* March 2017, vol. 19 (2